# EUROPEAN PATENT APPLICATION

(11) **EP 4 847 531 A1**
(43) Date of publication of application: **02.12.2026**
(21) Application number: 25745261.5
(22) Date of filing: 24.01.2025
(51) Int. Cl.: C07K 16/28, C07K 14/54, A61P 37/00, A61K 39/00

(54) **ANTI-TIGIT ANTIBODY SPECIFICALLY BINDING TO TIGIT, FUSION PROTEIN COMPRISING SAME AND INTERLEUKIN-10, AND USES THEREOF**

(30) Priority: 24.01.2024 KR 20240011242
(71) Applicant: MediMabbio Inc., Seongnam-si, Gyeonggi-do 13449 (KR)
(72) Inventor: KANG, Yuhoi, Seongnam-si, Gyeonggi-do 13449 (KR); JO, Hongseok, Seongnam-si, Gyeonggi-do 13449 (KR)
(74) Representative: Reddie & Grose LLP
(86) International application number: PCT/KR2025/001532
(87) International publication number: WO 2025/159595

(57) **Abstract**

An anti-TIGIT antibody or its antigen-binding fragment that specifically binds to TIGIT; a fusion protein comprising the antibody or antigen-binding fragment and interleukin-10 (IL-10); and a use of the antibody or antigen-binding fragment and/or fusion protein is provided.

## Description

### [Technical Field]

### Cross-Reference to Related Applications

This application claims the benefit of and priority to Korean Patent Application No. 10-2024-0011242 filed on January 24, 2024, the entire contents of which are incorporated herein by reference.

The present disclosure relates to an anti-TIGIT antibody or an antigen-binding fragment thereof that binds specifically to TIGIT, a fusion protein including the antibody or an antigen-binding fragment thereof and interleukin-10 (IL-10), and a use of the antibody or an antigen-binding fragment thereof and/or the fusion protein.

### [Background Art]

Autoimmune and anti-inflammatory immunotherapy is a method of treating autoimmunity and inflammation by altering the body's inappropriate immune response to alleviate related pathologies. Autoimmune immunotherapy involves mechanisms that suppress the immune system to reduce inappropriate recognition or response to healthy tissues. Immune system activation is a complex mechanism involving the functions of various cells, such as antigen-presenting cells, which are crucial for initiating antigen-specific responses, and effector cells, which are responsible for autoimmune and inflammation-related pathologies.

Representative examples of the above-mentioned effector cells include T cells and NK cells.

Meanwhile, TIGIT (T Cell immunoglobulin and ITIM (immune receptor tyrosine-based inhibitory motif) domain) is referred to as WUCAM, VSIG9, or Vstm3 Fig. and is a co-inhibitory receptor preferentially expressed in NK, CD8+, and CD4+ T Cells and regulatory T Cells ("Treg"). TIGIT is a transmembrane protein comprising an intracellular ITIM domain, a transmembrane domain, and an immunoglobulin variable domain.

TIGIT expression is increased in lymphocytes infiltrating tissues in disease environments such as autoimmunity, inflammation, and infection. TIGIT expression can serve as a marker for activated T cells that possess greater effector function compared to TIGIT-negative cells. Furthermore, TIGIT-expressing Treg cells exhibit enhanced immunosuppressive activity compared to the TIGIT-negative Treg population.

Based on this, drugs targeting TIGIT can selectively act on effector T and NK cells.

### [Detailed Description of the Invention]

### [Technical Problem]

One aspect provides an anti-TIGIT antibody or an antigen-binding fragment thereof that specifically recognizes TIGIT and/or specifically binds to TIGIT.

Another aspect provides a nucleic acid molecule encoding the anti-TIGIT antibody or an antigen-binding fragment thereof.

Another aspect provides a recombinant vector carrying the nucleic acid molecule encoding the anti-TIGIT antibody or an antigen-binding fragment thereof.

Another aspect provides a recombinant cell comprising the nucleic acid molecule or the recombinant vector.

Another aspect provides a composition for delivery of a biologically active substance (also referred to as a delivery vehicle for a biologically active substance or a delivery system for a biologically active substance), comprising at least one selected from the group consisting of the anti-TIGIT antibody or antigen-binding fragment thereof, the nucleic acid molecule, the recombinant vector, and the recombinant cell as described above. The composition for delivery of a biologically active substance may further comprise the biologically active substance to be delivered, in which case the biologically active substance may be conjugated to the antibody or antigen-binding fragment thereof, but not limited thereto.

Another aspect provides a method for preparing a composition for delivery of a biologically active substance, comprising a step of conjugating a biologically active substance to the anti-TIGIT antibody or antigen-binding fragment thereof. The conjugation may be performed recombinantly, chemically, or physically, but not limited thereto.

Another aspect provides a method for delivering a biologically active substance, comprising a step of administering the composition for delivery of a biologically active substance to a subject in need of delivery of the biologically active substance.

Another aspect provides a use of at least one selected from the group consisting of the anti-TIGIT antibody or antigen-binding fragment thereof, the nucleic acid molecule, the recombinant vector and the recombinant cell, for delivery of a biologically active substance and/or for preparing a composition for delivery of a biologically active substance.

Another aspect provides a composition for detecting TIGIT, comprising the anti-TIGIT antibody or antigen-binding fragment thereof.

Another aspect provides a method for detecting TIGIT, comprising: a step of contacting the anti-TIGIT antibody or antigen-binding fragment thereof with a biological sample; and a step of determining whether an antigen-antibody reaction (binding) has occurred.

Another aspect provides a use of the anti-TIGIT antibody or antigen-binding fragment thereof for detecting TIGIT.

Another aspect provides a composition for diagnosing a disease associated with TIGIT expression or overexpression, comprising the anti-TIGIT antibody or antigen-binding fragment thereof.

Another aspect provides a method for diagnosing a disease associated with TIGIT expression or overexpression, or a method for providing information for the diagnosis of the disease, comprising: a step of contacting the anti-TIGIT antibody or antigen-binding fragment thereof with a biological sample; and a step of determining whether an antigen-antibody reaction (binding) has occurred in the sample.

Another aspect provides a use of the anti-TIGIT antibody or antigen-binding fragment thereof for diagnosing a disease associated with TIGIT expression or overexpression.

Another aspect provides a fusion protein including: (1) an anti-TIGIT antibody or an antigen-binding fragment thereof; and (2) IL-10.

In the fusion protein according to an embodiment, IL-10 may be linked to a C-terminus and/or N-terminus of the heavy chain (heavy chain variable region or heavy chain constant region) or light chain (light chain variable region or light chain constant region) of the anti-TIGIT antibody or antigen-binding fragment thereof, either through a linker or directly without a linker.

Another aspect provides a nucleic acid molecule encoding the fusion protein.

Another aspect provides a recombinant vector comprising the nucleic acid molecule encoding the fusion protein.

Another aspect provides a recombinant cell comprising the nucleic acid molecule or the recombinant vector.

Another aspect provides an immunosuppressant agent or a pharmaceutical composition for immune suppression, comprising as an active ingredient at least one selected from the group consisting of the antibody or antigen-binding fragment thereof, the fusion protein, a nucleic acid molecule encoding the antibody or antigen-binding fragment thereof or the fusion protein, and a recombinant vector comprising the nucleic acid molecule, and a recombinant cell comprising the nucleic acid molecule or recombinant vector.

Another aspect provides a pharmaceutical composition for the prevention and/or treatment of immune-related diseases, comprising as an active ingredient at least one selected from the group consisting of the antibody or antigen-binding fragment thereof, the fusion protein, a nucleic acid molecule encoding the antibody or antigen-binding fragment thereof or the fusion protein, and a recombinant vector comprising the nucleic acid molecule, and a recombinant cell comprising the nucleic acid molecule or recombinant vector.

Another aspect provides a pharmaceutical composition for the prevention and/or treatment of autoimmune diseases and/or inflammatory diseases, comprising as an active ingredient at least one selected from the group consisting of the antibody or antigen-binding fragment thereof, the fusion protein, a nucleic acid molecule encoding the antibody or antigen-binding fragment thereof or the fusion protein, and a recombinant vector comprising the nucleic acid molecule, and a recombinant cell comprising the nucleic acid molecule or recombinant vector.

Another aspect provides a method of suppressing immunity, comprising a step of administering a pharmaceutically effective amount of at least one selected from the group consisting of the antibody or antigen-binding fragment thereof, the fusion protein, a nucleic acid molecule encoding the antibody or antigen-binding fragment thereof or the fusion protein, and a recombinant vector comprising the nucleic acid molecule, and a recombinant cell comprising the nucleic acid molecule or recombinant vector, to a subject in need of immune suppression.

Another aspect provides a method of the prevention and/or treatment of immune-related diseases, the method including a step of administering a pharmaceutically effective amount of at least one selected from the group consisting of the antibody or antigen-binding fragment thereof, the fusion protein, a nucleic acid molecule encoding the antibody or antigen-binding fragment thereof or the fusion protein, and a recombinant vector comprising the nucleic acid molecule, and a recombinant cell comprising the nucleic acid molecule or recombinant vector, to a subject in need of prevention and/or treatment of immune-related diseases.

Another aspect provides a method for the prevention and/or treatment of autoimmune diseases and/or inflammatory diseases, the method including a step of administering a pharmaceutically effective amount of at least one selected from the group consisting of the antibody or antigen-binding fragment thereof, the fusion protein, a nucleic acid molecule encoding the antibody or antigen-binding fragment thereof or the fusion protein, and a recombinant vector comprising the nucleic acid molecule, and a recombinant cell comprising the nucleic acid molecule or recombinant vector, to a subject in need of prevention and/or treatment of autoimmune diseases and/or inflammatory diseases.

Another aspect provides a use of at least one selected from the group consisting of the antibody or antigen-binding fragment thereof, the fusion protein, a nucleic acid molecule encoding the antibody or antigen-binding fragment thereof or the fusion protein, and a recombinant vector comprising the nucleic acid molecule, and a recombinant cell comprising the nucleic acid molecule or recombinant vector, for suppressing immunity or for the manufacture of immunosuppressant agents.

Another aspect provides a use of at least one selected from the group consisting of the antibody or antigen-binding fragment thereof, the fusion protein, a nucleic acid molecule encoding the antibody or antigen-binding fragment thereof or the fusion protein, and a recombinant vector comprising the nucleic acid molecule, and a recombinant cell comprising the nucleic acid molecule or recombinant vector, for the prevention and/or treatment of immune-related diseases or for the manufacture of drugs for the prevention and/or treatment of immune-related diseases.

Another aspect provides a use of at least one selected from the group consisting of the antibody or antigen-binding fragment thereof, the fusion protein, a nucleic acid molecule encoding the antibody or antigen-binding fragment thereof or the fusion protein, and a recombinant vector comprising the nucleic acid molecule, and a recombinant cell comprising the nucleic acid molecule or recombinant vector, for the prevention and/or treatment of autoimmune diseases and/or inflammatory diseases, or for the manufacture of drugs for the prevention and/or treatment of autoimmune diseases and/or inflammatory diseases.

### [Technical Solution]

The present application provides an anti-TIGIT antibody that binds to TIGIT or an antigen-binding fragment thereof; a fusion protein comprising (1) the anti-TIGIT antibody or the antigen-binding fragment thereof and (2) IL-10; and a pharmaceutical use thereof. The anti-TIGIT antibody or the antigen-binding fragment thereof has an excellent binding affinity to TIGIT and thus, it may have an activity of delivering other useful substances (e.g., IL-10) to a site where TIGIT is present, for example, a cell expressing (overexpressing) TIGIT on its surface. The fusion protein has the advantage of being applicable as various immunosuppressive agents and/or immunotherapeutic agents because (1) it suppresses immunity by targeting T cells and NK cells that have been activated due to the reasons such as increased TIGIT expression by means of an anti-TIGIT antibody or the antigen-binding fragment thereof, and (2) it has an excellent binding affinity to a receptor and low immunogenicity by means of IL-10.

Below, a detailed description will be given of the present disclosure.

### Definition of Terms

As used herein, the phrase that a nucleic acid molecule (which may be used interchangeably with "polynucleotide" or "gene") or a polypeptide (which may be used interchangeably with "protein") "comprises a specific nucleic acid sequence or amino acid sequence", "consists of a specific nucleic acid sequence or amino acid sequence", or "is represented by a specific nucleic acid sequence or amino acid sequence" means that the nucleic acid molecule or polypeptide essentially includes the specific nucleic acid sequence or amino acid sequence, and can be interpreted to include "substantially equivalent sequences" with variations (e.g., deletions, substitutions, modifications, and/or additions) in the specific nucleic acid sequence or amino acid sequence (or not excluding such variations from the specific nucleic acid sequence or amino acid sequence), as long as the original function and/or intended function of the nucleic acid molecule or polypeptide is maintained.

In an embodiment, the nucleic acid sequences or amino acid sequences provided herein may include mutants obtained by conventional mutagenesis methods, such as directed evolution or site-directed mutagenesis, insofar as the mutants retain the original function or desired function of the sequence. In an embodiment, when a nucleic acid molecule or polypeptide "comprises or consists of a specific nucleic acid sequence or amino acid sequence", this means that the nucleic acid molecule or polypeptide (i) essentially includes the specific nucleic acid sequence or amino acid sequence, or (ii) consists essentially of or essentially includes an amino acid sequence having 60% or greater, 70% or greater, 80% or greater, 85% or greater, 90% or greater, 91% or greater, 92% or greater, 93% or greater, 94% or greater, 95% or greater, 96% or greater, 97% or greater, 98% or greater, 99% or greater, 99.5% or greater, or 99.9% or greater homology with the specific nucleic acid sequence or amino acid sequence, while retaining its original function and/or desired function.

The nucleic acid sequences described herein may undergo various modifications within the coding region to reflect the codon preferences of the microorganism used for protein expression, as long as the amino acid sequence and/or function of the expressed protein remains unchanged due to the degeneracy of codons.

As used herein, the term "homology" refers to the degree of similarity between a given nucleic acid sequence or amino acid sequence and another sequence, and can be expressed as a percentage (%). Homology for nucleic acid sequences can be determined using well-known algorithms such as BLAST or FASTA, and programs like BLASTN or BLASTX have been developed based on the BLAST algorithm (see: http://www.ncbi.nlm.nih.gov).

### Anti-TIGIT antibody or antigen-binding fragment thereof

One embodiment of the present application provides an anti-TIGIT antibody or an antigen-binding fragment thereof, having an activity of specifically recognizing TIGIT and/or specifically binding to TIGIT.

The anti-TIGIT antibody or antigen-binding fragment thereof may include:
a polypeptide (CDR-H1) including the amino acid sequence of SEQ ID NO: 1,
a polypeptide (CDR-H2) including the amino acid sequence of SEQ ID NO: 2,
a polypeptide (CDR-H3) including the amino acid sequence of SEQ ID NO: 3,
a polypeptide (CDR-L1) including the amino acid sequence of SEQ ID NO: 4,
a polypeptide (CDR-L2) including the amino acid sequence of SEQ ID NO: 5, and
a polypeptide (CDR-L3) including the amino acid sequence of SEQ ID NO: 6.

Herein, the complementarity-determining regions (CDRs) are determined based on the CDR definitions according to the Kabat system.

In an embodiment, six CDRs (CDR-L1, CDR-L2, CDR-L3, CDR-H1, CDR-H2, and CDR-H3) that may be included in the anti-TIGIT antibody or antigen-binding fragment thereof are summarized in Table 1 below:

**[Table 1]**

| | Amino acid sequence | SEQ ID NO |
|---|---|---|
| CDR-H1 | SDYAWN | 1 |
| CDR-H2 | YITYSGSTSYNPSLKS | 2 |
| CDR-H3 | RQVGLGFAY | 3 |
| CDR-L1 | KASQDVSNTVA | 4 |
| CDR-L2 | SASYRYT | 5 |
| CDR-L3 | QQHYSTPWT | 6 |

(In Table 1, CDR-H1, CDR-H2 and CDR-H3 represent complementarity-determining regions on the heavy chain, while CDR-L1, CDR-L2, and CDR-L3 represent complementarity-determining regions on the light chain.)

In an embodiment, the anti-TIGIT antibody or antigen-binding fragment thereof may include:
a heavy chain variable region comprising CDR-H1 of SEQ ID NO: 1, CDR-H2 of SEQ ID NO: 2, and CDR-H3 of SEQ ID NO: 3; and
a light chain variable region comprising CDR-L1 of SEQ ID NO: 4, CDR-L2 of SEQ ID NO: 5 and CDR-L3 of SEQ ID NO:6.

More specifically, the anti-TIGIT antibody or antigen-binding fragment thereof may include:
a heavy chain variable region comprising an amino acid sequence selected from SEQ ID NO: 7 and SEQ ID NO: 29 to 34; and
a light chain variable region comprising an amino acid sequence selected from SEQ ID NO: 8 and SEQ ID NO: 35 to 41.

In some cases (e.g., when constructed recombinantly), the heavy chain variable region and/or light chain variable region may further include an appropriate signal sequence at the N-terminus thereof.

Table 2 below illustrates the amino acid sequences of heavy chain and light chain variable regions that may be included in the anti-TIGIT antibody or antigen-binding fragment thereof:

**[Table 2]**

| Heavy chain variable region | Amino acid sequence(N→C) | SEQ ID NO |
|---|---|---|
| VH0 | DVQLQESGPGLVKPSQSLSLTCTVTGYSITSDYA WNWIRQFPGNKLEWMAYITYSGSTSYNPSLKS RISITRDTSKNQFFLQLNSVTIEDTATYYCASRQ VGLGFAYWGQGTLVTVSA | 29 |
| VH1 | DVQLQESGPGLVKPSQTLSLTCTVTGYSITSDYA WNWIRQPPGKGLEWMAYITYSGSTSYNPSLKS RITISRDTSKNQFSLKLSSVTIEDTATYYCASRQ VGLGFAYWGQGTLVTVSS | 30 |
| VH6 | DVQLQESGPGLVKPSQTLSLTCTVTGYSITSDYA WNWIRQPPGKGLEWMAYITYSGSTSYNPSLKS RITISRDTSKNQFFLKLSSVTIEDTATYYCASRQ VGLGFAYWGQGTLVTVSS | 31 |
| VH7 | DVQLQESGPGLVKPSQTLSLTCTVTGYSITSDYA WNWIRQFPGNKLEWMAYITYSGSTSYNPSLKS RITISRDTSKNQFSLKLSSVTIEDTATYYCASRQ VGLGFAYWGQGTLVTVSS | 32 |
| VH5 | QVQLQESGPGLVKPSQTLSLTCTVTGYSITSDYA WNWIRQPPGKGLEWMAYITYSGSTSYNPSLKS RVTISVDTSKNQFSLKLSSVTAADTAVYYCASR QVGLGFAYWGQGTLVTVSS | 33 |
| VH8 | QVQLQESGPGLVKPSQTLSLTCTVTGYSITSDYA WNWIRQPPGKGLEWMAYITYSGSTSYNPSLKS RVTISVDTSKNQFFLKLSSVTAADTAVYYCASR QVGLGFAYWGQGTLVTVSS | 34 |
| VH9 | QVQLQESGPGLVKPSQTLSLTCTVTGYSITSDYA WNWIRQFPGNKLEWMAYITYSGSTSYNPSLKS RVTISVDTSKNQFSLKLSSVTAADTAVYYCASR QVGLGFAYWGQGTLVTVSS | 7 |

| Light chain variable region | Amino acid sequence (N→C) | SEQ ID NO |
|---|---|---|
| Vk0 | DIVMTQSHKFMSTSVGDRVSITCKASQDVSNT VAWYQQRPGQSPKPLIYSASYRYTGVPDRFTGS GSGTDFTFTISSVQAEDLAVYYCQQHYSTPWTF GGGTKLEIK | 35 |
| Vk1 | DIVMTQSHSFLSASVGDRVSITCKASQDVSNTV AWYQQKPGQPPKPLIYSASYRYTGVPDRFTGS GSGTDFTLTISSLQAEDVAVYYCQQHYSTPWTF GGGTKVEIK | 36 |
| Vk2 | DIVMTQSPSSLSASVGDRVSITCKASQDVSNTV AWYQQKPGQPPKPLIYSASYRYTGVPDRFTGS GSGTDFTLTISSLQAEDVAVYYCQQHYSTPWTF GGGTKVEIK | 37 |
| Vk7 | DIVMTQSPSSLSASVGDRVSITCKASQDVSNTV AWYQQKPGQAPRPLIYSASYRYTGVPDRFTGS GSGTDFTLTISSLQAEDVAVYYCQQHYSTPWTF GGGTKVEIK | 38 |
| Vk11 | DIVMTQSHSSLSASVGDRVSITCKASQDVSNTV AWYQQKPGQPPKPLIYSASYRYTGVPDRFTGS GSGTDFTLTISSLQAEDVAVYYCQQHYSTPWTF GGGTKVEIK | 8 |
| Vk5 | DIQMTQSPSSLSASVGDRVSITCKASQDVSNTV AWYQQKPGQPPKPLIYSASYRYTGVPDRFSGSG SGTDFTLTISSLQPEDVATYYCQQHYSTPWTFG GGTKVEIK | 39 |
| Vk10 | DIQMTQSPSSLSASVGDRVSITCKASQDVSNTV AWYQQKPGQAPRPLIYSASYRYTGVPDRFSGS GSGTDFTLTISSLQPEDVATYYCQQHYSTPWTF GGGTKVEIK | 40 |
| Vk14 | DIQMTQSPSFLSASVGDRVSITCKASQDVSNTV AWYQQKPGQPPKPLIYSASYRYTGVPDRFSGSG SGTDFTLTISSLQPEDVATYYCQQHYSTPWTFG GGTKVEIK | 41 |

(In Table 2, the underlined regions sequentially represent the CDR1, CDR2, and CDR3 of the heavy and light chains.)

In one example, the anti-TIGIT antibody or its antigen-binding fragment provided herein is for (binding to/recognizing) a TIGIT protein, e.g., a human TIGIT protein (NCBI Reference Sequence NP_776160.2; UniProtKB/SwissProt Q495A1-1), and may bind to one or more or two or more amino acids (e.g., located consecutively) within the protein, but is not limited thereto.

### [Human TIGIT protein (UniProtKB/SwissProt Q495A1-1)]

1 MRWCLLLIWA QGLRQAPLAS GMMTGTIETT GNISAEKGGS
IILQCHLSST TAQVTQVNWE
61 QQDQLLAICN ADLGWHISPS FKDRVAPGPG LGLTLQSLTV
NDTGEYFCIY HTYPDGTYTG
121 RIFLEVLESS VAEHGARFQI PLLGAMAATL VVICTAVIVV
VALTRKKKAL RIHSVEGDLR
181 RKSAGQEEWS PSAPSPPGSC VQAEAAPAGL CGEQRGEDCA
ELHDYFNVLS YRSLGNCSFF
241 TETG (SEQ ID N O: 42)

As used herein, the term "antibody" refers to a protein that specifically binds to a particular antigen. This protein can be produced in response to antigen stimulation within the immune system or can be chemically synthesized or recombinantly produced, with no limitations to the types thereof. The antibody may be one that is non-naturally produced, for example, recombinantly or synthetically generated. The antibody may be an animal antibody (e.g., a mouse antibody), a chimeric antibody, a humanized antibody, or a human antibody. The antibody may be a monoclonal or polyclonal antibody.

In the anti-TIGIT antibody or antigen-binding fragment thereof provided herein, the regions other than the defined heavy chain CDR and light chain CDR regions, or the heavy chain variable region and light chain variable region, may be derived from immunoglobulins of any subtype (e.g., IgA, IgD, IgE, IgG (IgG1, IgG2, IgG3, or IgG4), IgM, etc.). For example, the remaining regions may be derived from the framework regions of any immunoglobulin subtype and/or from the constant regions of the light and/or heavy chains. In an embodiment, the anti-TIGIT antibody provided herein may be a human IgG-type antibody, for example, in the form of IgG1, IgG2, IgG3, or IgG4, but with no limitations thereto.

An intact antibody (e.g., IgG type) has a structure comprising two full-length light chains and two full-length heavy chains, with each light chain linked to a heavy chain via a disulfide bond. The constant region of the antibody is divided into a heavy chain constant region and a light chain constant region. The heavy chain constant region has gamma (γ), mu (µ), alpha (α), delta (δ), and epsilon (ε) types, with subclasses including gamma 1 (γ1), gamma 2 (γ2), gamma 3 (γ3), gamma 4 (γ4), alpha1 (α1), and alpha 2 (α2). The light chain constant region has kappa (κ) and lambda (λ) types.

In an embodiment, the anti-TIGIT antibody provided herein may include an IgG constant region as the heavy chain constant region and a kappa constant region as the light chain constant region, but is not limited thereto.

In an embodiment, the constant region of the IgG (e.g., human IgG1) may be wild-type. In another embodiment, the constant region of the IgG may be a variant including one or more mutations selected from the group consisting of S240D (substitution of serine at position 240 with aspartic acid), A331L, I333E, N298A, S299A, E334A, K335A, L235A, L236A, P330G, and K478 deletion (ΔK478), and may include, for example, the following mutations:
(1) S240D, A331L, and I333E;
(2) N298A;
(3) S299A, E334A, and K335A;
(4) L235A, L236A, and P330G; or
(5) K478 deletion.

The term "heavy chain" is construed to encompass both a full-length heavy chain and a fragment thereof that includes a variable region domain (VH) containing an amino acid sequence sufficient to impart antigen specificity, and three constant region domains CH1, CH2, and CH3, along with a hinge. The term "light chain" is construed to encompass both a full-length light chain and a fragment thereof that includes a variable region domain VL containing an amino acid sequence sufficient to impart antigen specificity, and a constant region domain CL.

The term "complementarity determining region" (CDR)" refers to a region of an antibody that confers antigen-binding specificity, comprising the amino acid sequences in the hypervariable regions of the heavy and light chains of immunoglobulins. The heavy chain and light chain each may contain three CDRs (CDRH1, CDRH2, CDRH3, and CDRL1, CDRL2, CDRL3). These CDRs may provide key contact residues for the antibody's binding to an antigen or epitope. As used herein, the term "specifically binds" or "specifically recognizes" refers to the interaction between an antigen and antibody that results in an immunological response, as is conventionally known in the art.

The complementarity-determining regions (CDRs) described herein are defined based on the Kabat system.

Unless otherwise specified, the term "antibody", as used herein, can be understood to encompass antigen-binding fragments of antibodies that retain antigen-binding ability.

The term "antigen-binding fragment" refers to any form of polypeptide that includes a portion capable of binding to an antigen (e.g., the six CDRs defined herein). Examples include scFv, scFv-Fc, (scFv)₂, Fab, Fab', or F(ab')₂, but are not limited thereto.

Among the antigen-binding fragments, Fab has a structure that includes the variable regions of the light and heavy chains, the constant region of the light chain, and the first constant region (CH1) of the heavy chain, having a single antigen-binding site.

Fab' differs from Fab in that the former includes a hinge region containing one or more cysteine residues at the C-terminus of the CH1 domain of the heavy chain.

F(ab')₂ antibodies are produced when the cysteine residues in the hinge region of Fab' form disulfide bonds. Fv is the smallest antibody fragment, including only the variable regions of the heavy and light chains, and recombinant techniques for generating Fv fragments are well known in the art.

Two-chain Fv includes a variable region of the heavy chain and a variable region of the light chain, with a non-covalent linkage therebetween, while single-chain Fv (scFv) includes a variable region of the light chain and a variable region of the heavy chain, which may be covalently bonded to each other generally via a peptide linker or directly at the C-terminus, forming a dimer-like structure similar to two-chain Fv.

The antigen-binding fragment may be obtained using proteolytic enzymes (e.g., Fab can be obtained by restrictive digestion of the whole antibody with papain, while F(ab')₂ fragments can be obtained by digestion with pepsin) or can be produced through recombinant DNA technology.

The term "hinge region" refers to a region included in the heavy chain of an antibody, located between the CH1 and CH2 domains, which functions to provide flexibility to the antigen-binding site within the antibody.

The antibody provided herein may be a monoclonal antibody. Monoclonal antibodies may be produced by methods well known in the art. For example, they can be produced using phage display techniques or can be generated as monoclonal antibodies derived from animals (e.g., mice) using conventional methods.

Individual monoclonal antibodies may be screened based on their ability to bind to the receptor-binding domain of TIGIT using standard ELISA (Enzyme-Linked ImmunoSorbent Assay) formats. Functional assays such as competitive ELISA or cell-based assays can be used to assess molecular interactions and inhibitory activity. The affinity (Kd values) of each monoclonal antibody for the receptor-binding domain of TIGIT can then be evaluated based on the selected antibodies with strong inhibitory activity.

The finally selected antibodies can be manufactured and used not only as antibodies in which the region other than the antigen-binding site has been converted into human immunoglobulin antibodies, but also as humanized antibodies. The method for manufacturing humanized antibodies is well known in the art.

The antigen-binding fragments of the anti-TIGIT antibody provided herein refer to fragments derived from the anti-TIGIT antibody that retain binding affinity for the antigen (TIGIT), and may be any polypeptide comprising the six CDRs of the anti-TIGIT antibody described above, such as scFv, scFv-Fc, scFv-Ck (kappa constant region), scFv-Cλ (lambda constant region), (scFv)₂, Fab, Fab', or F(ab')₂, but with no limitations thereto. In an embodiment, the antigen-binding fragment may be scFv, or a fusion polypeptide in which scFv is fused to the Fc region of an immunoglobulin (e.g., IgG1, IgG2, IgG3, IgG4, etc.) (scFv-Fc) or to the constant region of a light chain (e.g., kappa or lambda) (scFv-Ck or scFv-Cλ), but is not limited thereto.

As used herein, the phrase that an antibody (e.g., CDR, variable region, or heavy/light chain, antigen-binding fragment, etc.) "comprises a specific amino acid sequence", or "represents or consists of a specific amino acid sequence" covers not only the case where the antibody necessarily includes the specific amino acid sequence, but also the case where modifications (e.g., substitutions, deletions, and/or additions of amino acid residues) that do not significantly affect the antibody's activity (e.g., antigen affinity, pharmacological activity, etc.) are introduced into the amino acid sequence.

The anti-TIGIT antibody or antigen-binding fragment provided herein may have a binding affinity (KD) for TIGIT (e.g., human TIGIT) of, for example, for example, 10mM or lower, 5mM or lower, 1mM or lower, 0.5mM or lower, 0.2mM or lower, 0.1mM or lower, 0.05mM or lower, 0.01mM or lower, 0.005mM or lower, or 0.001 mM or lower, as measured by surface plasmon resonance (SPR). Specifically, the KD may be within a range of 0.0001nM to 10mM, 0.0005nM to 10mM, 0.001nM to 10mM, 0.005nM to 10mM, 0.01nM to 10mM, 0.05nM to 10mM, 0.1nM to 10mM, 0.5nM to 10mM, 1nM to 10mM, 0.0001nM to 5mM, 0.0005nM to 5mM, 0.001nM to 5mM, 0.005nM to 5mM, 0.01nM to 5mM, 0.05nM to 5mM, 0.05nM to 5mM, 0.1nM to 5mM, 0.5nM to 5mM, 1nM to 5mM, 0.0001nM to 1mM, 0.0005nM to 1mM, 0.001nM to 1mM, 0.005nM to 1mM, 0.01nM to 1mM, 0.05nM to 1mM, 0.1nM to 1mM, 0.5nM to 1mM, 1nM to 1mM, 0.0001nM to 0.5mM, 0.0005nM to 0.5mM, 0.001nM to 0.5mM, 0.005nM to 0.5mM, 0.01nM to 0.5mM, 0.05nM to 0.5mM, 0.05nM to 0.5mM, 0.1nM to 0.5mM, 0.5nM to 0.5mM, 1nM to 0.5mM, 0.0001nM to 0.2mM, 0.0005nM to 0.2mM, 0.001nM to 0.2mM, 0.005nM to 0.2mM, 0.01nM to 0.2mM, 0.05nM to 0.2mM, 0.1nM to 0.2mM, 0.5nM to 0.2mM, 1nM to 0.2mM, 0.0001nM to 0.1mM, 0.0005nM to 0.1mM, 0.001nM to 0.1mM, 0.005nM to 0.1mM, 0.01nM to 0.1mM, 0.01nM to 0.1mM, 0.05nM to 0.1mM, 0.1nM to 0.1mM, 0.5nM to 0.1mM, 1nM to 0.1mM, 0.0001nM to 0.05mM, 0.0005nM to 0.05mM, 0.001nM to 0.05mM, 0.005nM to 0.05mM, 0.01nM to 0.05mM, 0.05nM to 0.05mM, 0.1nM to 0.05mM, 0.5nM to 0.05mM, 1nM to 0.05mM, 0.0001nM to 0.01mM, 0.0005nM to 0.01mM, 0.001nM to 0.01mM, 0.001nM to 0.01mM, 0.005nM to 0.01mM, 0.005nM to 0.01mM It may be 0.01mM, 0.01nM to 0.01mM, 0.05nM to 0.01mM, 0.1nM to 0.01mM, 0.5nM to 0.01mM, or 1nM to 0.01mM, but is not limited thereto.

Another embodiment of the present application provides a nucleic acid molecule encoding the anti-TIGIT antibody or an antigen-binding fragment thereof.

Another embodiment provides a recombinant vector including the nucleic acid molecule encoding the anti-TIGIT antibody or an antigen-binding fragment thereof.

Another embodiment provides a recombinant cell comprising the nucleic acid molecule or the recombinant vector.

The anti-TIGIT antibody or an antigen-binding fragment thereof has the characteristic of having excellent binding affinity to TIGIT and not inhibiting interaction (binding) with proteins that interact with TIGIT (e.g., CD155). Due to this characteristic, the anti-TIGIT antibody or its antigen-binding fragment can bind (fuse/conjugate) with a biologically active substance (e.g., IL-10) that has a desired effect and have the activity of effectively delivering the biologically active substance to a site where TIGIT is present, such as a cell (e.g., immune cell such as a T cell or NK (natural killer) cell) that expresses (overexpresses) TIGIT on its surface, without affecting the function/activity/action of TIGIT.

Accordingly, another embodiment of the present application provides a composition for delivering a biologically active substance (delivery vehicle for biologically active substance or delivery system for biologically active substance) comprising one or more selected from the group consisting of the above-mentioned antibody or antigen binding fragment thereof, nucleic acid molecule, recombinant vector, and recombinant cell. The composition for delivering a biologically active substance may further include the biologically active substance to be delivered. In this case, the biologically active substance may be connected to the antibody or antigen binding fragment (in the form of an antibody-biologically active substance conjugate; e.g., an antibody-drug conjugate (ADC), etc.), but is not limited thereto.

Other embodiment provides a method for preparing a composition for delivering a biologically active substance, comprising the step of binding a biologically active substance to the antibody or antigen binding fragment. The binding may be performed recombinantly, chemically, or physically, but is not limited thereto.

Other embodiment provides a method for delivering a biologically active substance, comprising a step of administering the above-described composition for delivering the biologically active substance, to a subject in need of delivery of the biologically active substance.

Other embodiment provides a use for delivering one or more biologically active substances selected from the group consisting of the above-mentioned antibody or antigen binding fragment, nucleic acid molecule, recombinant vector, and recombinant cell, and/or a use for preparing a composition for delivering biologically active substances.

The biologically active substance may be one or more selected from the group consisting of all biocompatible substances such as a small molecule compound, protein, peptide, nucleic acid molecule (e.g., antisense oligonucleotide, siRNA, shRNA, microRNA, aptamer, etc.), and labeling substances such as radioactive isotopes, in which they are capable of exhibiting beneficial or desired effects, such as immunosuppression, immune activation, visualization, etc., at a site delivered by the above-mentioned anti-TIGIT antibody or its antigen binding fragment, such as a cell (e.g., immune cell such as a T cell, NK (natural killer) cell) that expresses (overexpresses) TIGIT on its surface.

For example, the biological active substance may be one or more selected from the group consisting of chemical drugs having an immunosuppressive or immunoactivating effect in immune cells; proteins having an immunosuppressive or immunoactivating effect in immune cells (e.g., cytokines, chemokines, etc.); ligands that bind to receptors on the surface of immune cells; antibodies that bind to immune-related proteins (receptors on the surface of immune cells, ligands that bind thereto, etc.), but is not limited thereto.

Other embodiment provides a composition for detecting TIGIT comprising the above-mentioned antibody or antigen-binding fragment. Another embodiment provides a method for detecting TIGIT comprising the steps of: treating a biological sample with the above-mentioned anti-TIGIT antibody or its antigen-binding fragment; and identifying whether an antigen-antibody reaction (binding) occurs. In the detection method, if an antigen-antibody reaction is detected, it can be determined that TIGIT is present in the biological sample. Another embodiment provides a use of the above-mentioned anti-TIGIT antibody or its antigen-binding fragment for detecting TIGIT. The detection of TIGIT means identifying the presence or expression of TIGIT in a biological sample, or the degree of the presence or expression of TIGIT.

The anti-TIGIT antibody or its antigen-binding fragment may be conjugated with a detectable label. If the detectable label is visible, the anti-TIGIT antibody or its antigen-binding fragment may be used for visualizing TIGIT in a biological sample. In one example, the detectable label may be one or more selected from the group consisting of radioactive isotopes (e.g., 3H, 14C, 35S, 90Y, 99Tc, 111In, 1251, 1311, 177Lu, 166Ho, 153Sm, etc.), enzymes, fluorescent labels, luminescent labels (e.g., bioluminescent labels), magnetic labels, and chemical substances such as biotin, but is not limited thereto.

The biological sample may be selected from a group consisting of cells (e.g., immune cells such as T cells, NK cells), tissues, and body fluids (e.g., blood, serum, etc.) obtained from mammals such as humans, monkeys, primates, mice, rats, etc., and may be isolated from a living organism.

Other embodiment provides a composition for diagnosing diseases related to TIGIT expression or overexpression, comprising the above-mentioned anti-TIGIT antibody or its antigen binding fragment.

Other embodiment provides a method for diagnosing a disease related to TIGIT expression or overexpression, or a method for providing information for diagnosing a disease, comprising the steps of contacting the anti-TIGIT antibody or its antigen-binding fragment with a biological sample and identifying whether an antigen-antibody reaction (binding) occurs within the sample.

Other embodiment provides a use of the anti-TIGIT antibody or its antigen binding fragment for diagnosing a disease related to TIGIT expression or overexpression.

The diseases related with the TIGIT expression or overexpression may be any disease caused by TIGIT expression or overexpression, for example, an autoimmune disease. The autoimmune disease includes, but is not limited to, rheumatoid arthritis, type 1 diabetes, Crohn's disease, ulcerative colitis, Behcet's syndrome, lupus (systemic lupus erythematosus; SLE), and Sjögren's syndrome, and may be, but is not limited to, one or more autoimmune diseases selected from the group consisting of myasthenia gravis, scleroderma, hypothyroidism, hyperthyroidism, psoriasis, vitiligo, multiple sclerosis, autoimmune hepatitis, autoimmune nephritis, autoimmune pancreatitis, autoimmune encephalitis, cytokine storm, and etc.

In the diagnostic method, the biological sample may be selected from a group consisting of cells (e.g., immune cells such as T cells, NK cells), tissues, and body fluids (e.g., blood, serum, etc.) obtained from mammals such as primates including humans and monkeys, and rodent including mice and rats, etc., which require diagnosis of diseases related to TIGIT expression or overexpression, and may be isolated from a living organism.

The step of identifying whether the antigen-antibody reaction occurs can be performed through various methods known in the art. For example, it can be measured through conventional enzyme reactions, fluorescence, luminescence, and/or radiation detection. Specifically, it can be measured by a method selected from the group consisting of, but not limited to, immunochromatography, immunohistochemistry, enzyme-linked immunosorbent assay (ELISA), radioimmunoassay (RIA), enzyme immunoassay (EIA), fluorescence immunoassay (FIA), luminescence immunoassay (LIA), Western blotting, and microarrays.

### Fusion Protein

In the present disclosure, an embodiment provides a fusion protein including: (1) an anti-TIGIT antibody or an antigen-binding fragment thereof that specifically recognizes TIGIT; and (2) interleukine-10 (IL-10).

The anti-TIGIT antibody or its antigen-binding fragment is as described above.

The fusion protein may have both functions of the anti-TIGIT antibody or its antigen-binding fragment (TIGIT recognition/binding, targeting, activation, etc.) and the functions of IL-10 (immunosuppression, recognition/binding of the IL-10 receptor (IL-10R), etc.).

The interleukin-10 (IL-10) may be selected from the group consisting of:
(a) a polypeptide including IL-10, and
(b) a polypeptide having a homology of 60% or more, 70% or more, 80% or more, 85% or more, 90% or more, 91% or more, 92% or more, 93% or more, 94% or more, 95% or more, 96% or more, 97% or more, 98% or more, or 99% or more with the polypeptide (a).

Interleukin-10 (IL-10), also known as human cytokine synthesis inhibitory factor (CSIF), is encoded by the gene IL-10 and is involved in signal transduction by binding to two IL-10Rα (also known as IL-10R1) and two IL-10Rβ (also known as IL-10R2). For example, interleukin-10 may be of human origin, such as NCBI Accession No. NP_000563.1 (amino acid residues 1-18 for signal sequence; amino acid residues 19-178 for mature segment), but is not limited thereto.

In one embodiment, the IL-10 may be represented by the following amino acid sequence (from N-terminus to C-terminus):

SPGQGTQSENSCTHFPGNLPNMLRDLRDAFSRVKTFFQMKDQLDNLLLK

ESLLEDFKGYLGCQALSEMIQFYLEEVMPQAENQDPDIKAHVNSLGENLKTLRL
RLRRCHRFLPCENKSKAVEQVKNAFNKLQEKGIYKAMSEFDIFINYIEAYMTMK
IRN (wild-type: SEQ ID NO: 9)

The polypeptide (a) including IL-10 may be in the form of a monomer containing one IL-10 or a polymer in which two or more IL-10s are linked (e.g., a dimer in which two IL-10s are linked). When IL-10 is in the form of a polymer (e.g., a dimer), two or more (e.g., two) IL-10 monomers may be connected through a linker or directly without a linker. When IL-10 is connected through a linker, the linker (second linker) may be selected from general peptide linkers, such as GGGGSGGG (SEQ ID NO. 16), but is not limited thereto.

The polypeptide (a) may include an additional oligopeptide for additional purposes, for example, to facilitate protein purification, such as a tag for labeling at the N-terminus (e.g., MHSSALLCCLVLLTGVRA (SEQ ID No. 45), etc.), but with no limitations thereto.

The interleukin-10 is characterized by low immunogenicity when administered to the human body due to its human origin.

The IL-10 may inhibit binding activity to its receptor, for example IL-10 receptor alpha-chain (IL10RA, CD210, CD210a, CDW210A, HIL-10R, IL-10R1, IL10R, Interleukin 10 receptor, alpha subunit, interleukin 10 receptor subunit alpha) , and /or IL-10 receptor beta-chain (IL10RB, CDW210B, CRF2-4, CRFB4, D21S58, D21S66, IL-10R2, Interleukin 10 receptor, beta subnunit, Interleukin 10 receptor subunit beta), and/or inhibit cytokine production and/or secretion activity in immune cells (e.g., T cells (CD4+, CD8+), NK cells, etc.).

In one embodiment, the IL-10 may be selected from the group consisting of:
(a) (a-1) a polypeptide (monomer) containing one amino acid sequence of SEQ ID NO. 9;
(a-2) a polypeptide (dimer) containing two amino acid sequences of SEQ ID NO. 9; and
(b) a polypeptide having 93% or more, 94% or more, 95% or more, 96% or more, 97% or more, 98% or more, or 99% or more homology with the polypeptide (a), and inhibiting/inducing binding activity to an IL-10 receptor and/or cytokine production and/or secretion activity in immune cells.

The polypeptide of (a-2) may be one in which two amino acid sequences of SEQ ID NO. 9 are connected with or without a peptide linker (e.g., SEQ ID NO. 16, etc.), but is not limited thereto.

The IL-10 may have activities such as binding activity to an IL-10 receptor, and/or inhibition of cytokine production and/or secretion in immune cells, and/or inhibition of cytokine production and/or secretion in non-immune cells (e.g., tissue cells).

In the fusion protein, the anti-TIGIT antibody or its antigen-binding fragment may have the function of delivering IL-10 to effector cells (e.g., effector T cells and/or Treg cells) and/or NK cells. Additionally, by maintaining the natural sequence of the antibody, it enables binding to the Fc receptor. Thus, the anti-TIGIT antibody can also target TIGIT-negative effector cells important for autoimmunity and/or inflammation, including monocytes and macrophages. The IL-10 is a safe polypeptide with good receptor binding affinity and low immunogenicity; When administered in vivo, it exhibits excellent immunosuppressive effects while having a low risk of inducing an immune response recognized as an immunogen. Therefore, the fusion protein can be utilized as various immunosuppressant agents and immunotherapeutic agents.

In the fusion protein according to an embodiment, IL-10 may be linked to the C-terminus and/or N-terminus of a heavy chain (heavy chain variable region or heavy chain constant region) or light chain (light chain variable region or light chain constant region) of the anti-TIGIT antibody or antigen-binding fragment thereof, either through a linker or directly without a linker, as a monomer or dimer.

More specifically, the fusion protein may include: (1) an anti-TIGIT antibody (e.g., an IgG type antibody including two heavy chains and two light chains) and (2) one IL-10, where the IL-10 is linked, either through a peptide linker or directly without a peptide linker, to the C-terminus (constant region C-terminus) or N-terminus of either of the two heavy chains or either of the two light chains of the anti-TIGIT antibody.

In another embodiment, the fusion protein may include: (1) an anti-TIGIT antibody (e.g., an IgG type antibody including two heavy chains and two light chains); and (2) two IL-10 molecules, where each IL-10 is linked, either through a linker or directly without a linker, to the C-terminus or N-terminus of either of the two heavy chains or either of the two light chains of the anti-TIGIT antibody.

For instance, the fusion protein may include:
(i) one of the two IL-10 molecules may be linked, either through a linker or directly without a linker, to the C-terminus (i.e., the C-terminus of the constant region (e.g., Fc)) or N-terminus (i.e., the N-terminus of the variable region) of one of the two heavy chains of the anti-TIGIT antibody, and the other IL-10 molecule may be linked, either through a linker or directly without a linker, to the C-terminus (i.e., the C-terminus of the constant region (e.g., Fc)) or N-terminus (i.e., the N-terminus of the variable region) of the other heavy chain of the anti-TIGIT antibody, or
(ii) one of the two IL-10 molecules may be linked, either through a linker or directly without a linker, to the C-terminus (i.e., the C-terminus of the constant region) or N-terminus (i.e., the N-terminus of the variable region) of one of the two light chains of the anti-TIGIT antibody, and the other IL-10 molecule may be linked, either through a linker or directly without a linker, to the C-terminus (i.e., the C-terminus of the constant region) or N-terminus (i.e., the N-terminus of the variable region) of the other light chain of the anti-TIGIT antibody.

More specifically, in the fusion protein:
(i) one of the two IL-10 molecules may be linked, either through a linker or directly without a linker, to the C-terminus of one Fc region of the two heavy chains of the anti-TIGIT antibody, and the other may be linked, either through a linker or directly without a linker, to the C-terminus of the other Fc region of the anti-TIGIT antibody, or
(ii) one of the two IL-10 molecules may be linked, either through a linker or directly without a linker, to the C-terminus of one of the two light chains, and the other may be linked, either through a linker or directly without a linker, to the C-terminus of the other light chain of the anti-TIGIT antibody.

The linker for connecting the antibody and IL-10 may be a rigid peptide linker (e.g., [EAAAK]n, wherein n is an integer of 1 to 5 representing the number of [EAAAK]) , a flexible peptide linker (e.g., [(G)mS]l, wherein m is an integer of 1 to 5 representing the number of G, and l is an integer of 1 to 5 representing the number of [(G)mS]), but is not limited thereto.

### Linker

The linker (first linker) for connecting the antibody and IL-10 may be a peptide linker, for example, at least one selected from the group consisting of [EAAAK]n (n is an integer from 1 to 5 representing the number of [EAAAK] (SEQ ID NO: 77)), [(G)mS]l (m is an integer from 1 to 5 representing the number of G residues, and l is an integer from 1 to 5 representing the number of [(G)mS]), A EAAAK A, A EAAAK EAAAK A, A EAAAK EAAAK EAAAK EAAAK A, A EAAAK EAAAK EAAAK EAAAK EAAAK A, A EAAAK EAAAK EAAAK EAAAK A, AEAAAKEAAAAKAG, AEAAAKEAAAAKAG, GGGG, GGGGG, GGGGGGG, GAGAGAGAGA, RPLSYRPPPPPFFPFGFPSVRP, YPRSYIRRHHPSSLTT, TPSHLSHILPSFGLPTFWN, RPVSPFTFPPRLSNSWLPA, SPAAHPRSIPRPPGPIRT, APGPSAPSHRSLPSRAAFG, PRNSHFLHPLLVAPLGA, MPSLSGVLQVRYLSPPDL, SPQYPSPLTLTLPPHPSL, NPSLNPPSYLHRAPSRIS, LPWRTSLPLPRRPP, PPLFAKGPVGLLSRSSFPP, VPPAPVVSSLRSAHARPPY, LRPTPPRVRSYTCCPP, PNVAHVLPLL TVPWDNLR, CNPLLPLCARSPAVRTFP, LGTPTPTPTTGEF, EDFTRGKL, L EAAAR EAAAR EAAAR EAAAR EAAAR EAAAR EAAAR, L EAAAR EAAAR EAAAR EAAR EAAR, L EAAR, L EAAR, EAAAR EAAAR EAAAR EAAAR, EAAAR EAAAR EAAAR, EAAAR, LTEEQQEGGG, TEEQQEGGG, LAKLKQKTEQLQDRIAGGG, LELKTPLGDT THTCPRCPEP KSCDTPPPCP RCPEPKSCDT PPPCPRCPEP KSCDTPPPCP RCPGG, and LEPKSSDKTHTSPPSPGG but are not limited thereto. For example, the peptide linker may be a rigid linker, such as [EAAAK]n (where n is an integer from 1 to 5 representing the number of [EAAAK], e.g., SEQ ID Nos:14, 46 to 49, etc.), or a flexible linker, such as [(G)mS]l (where m is an integer from 1 to 5 representing the number of G residues, and l is an integer from 1 to 5 representing the number of [(G)mS], e.g., SEQ ID Nos: 15, 98 to 101, etc), but is not limited thereto.

### Nucleic Acid Molecules, Recombinant Vectors, and Recombinant Cells

Another embodiment provides a nucleic acid molecule encoding an antibody, antigen-binding fragment, or the fusion protein.

Another embodiment provides a recombinant vector carrying the nucleic acid molecule. The vector may be used as an expression vector for the nucleic acid molecule.

Another embodiment provides a recombinant cell anchoring the nucleic acid molecule or a recombinant vector including the same. The cell may be used for the production of the fusion protein.

Another embodiment provides a method for producing the fusion protein, the method comprising a step of expressing the nucleic acid molecule in an appropriate host cell. The step of expressing the nucleic acid molecule in an appropriate host cell may include culturing the recombinant cell described above under a standard condition.

The term "vector" refers to a means for expressing a target gene in a host cell. Examples of the vectors include plasmid vectors, cosmid vectors, and viral vectors such as bacteriophage vectors, adenovirus vectors, retrovirus vectors, and adeno-associated virus vectors. Vectors that may be used as recombinant vectors include commonly used plasmids in the art (e.g., pSC101, pGV1106, pACYC177, ColE1, pKT230, pME290, pBR322, pUC8/9, pUC6, pBD9, pHC79, pIJ61, pLAFR1, pHV14, pGEX series, pET series, and pUC19), phages (e.g., λgt4λB, λ-Charon, λΔz1, and M13), or viruses (e.g., SV40).

In the recombinant vector, the nucleic acid molecule encoding the fusion protein may be operatively linked to a promoter. The term "operatively linked" refers to a functional connection between a nucleotide regulatory sequence (e.g., a promoter sequence) and another nucleotide sequence. The regulatory sequence, when "operatively linked", controls the transcription and/or translation of the other nucleotide sequence.

The recombinant vector may typically be constructed as a cloning vector or an expression vector. The expression vector may be any conventional vector used in the art for expressing foreign proteins in plants, animals, or microorganisms. The recombinant vector may be constructed using various methods known in the art.

The recombinant vector may be constructed using prokaryotic or eukaryotic cells as a host. For example, when the vector is an expression vector and prokaryotic cells are used as the host, it is common for the vector to include a strong promoter capable of driving transcription (e.g., pLλ promoter, CMV promoter, trp promoter, lac promoter, tac promoter, T7 promoter, etc.), a ribosome binding site for initiating translation, and transcription/translation termination sequences. When eukaryotic cells are used as the host, the vector may include eukaryotic cell-origin replication origins such as f1 origin, SV40 origin, pMB1 origin, adenovirus origin, AAV origin, and BBV origin, but is not limited thereto. Additionally, the vector may include a promoter derived from a mammalian genome (e.g., metallothionein promoter) or from a mammalian virus (e.g., adenovirus late promoter, vaccinia virus 7.5K promoter, SV40 promoter, cytomegalovirus promoter, and HSV tk promoter), and typically includes a polyadenylation sequence as a transcription termination sequence.

The recombinant cell may be obtained by introducing the recombinant vector into an appropriate host cell. So long as it is known in the art, any host cell that can stably and continuously clone or express the recombinant vector may be available. Prokaryotic host cells may include, for example, E. coli JM109, E. coli BL21, E. coli RR1, E. coli LE392, E. coli B, E. coli X 1776, E. coli W3110, Bacillus spp. such as Bacillus subtilis and Bacillus thuringiensis, and gut bacteria such as Salmonella typhimurium, Serratia marcescens, and various Pseudomonas species. For eukaryotic host cells, transformed host cells may include yeast (Saccharomyces cerevisiae), insect cells, plant cells, and animal cells, for example, Sp2/0, CHO (Chinese hamster ovary) K1, CHO, DG44, PER.C6, W138, BHK, COS-7, 293, HepG2, Huh7, 3T3, RIN, MDCK cell lines, but are not limited thereto.

The delivery (introduction) of the nucleic acid molecule or recombinant vector into the host cell may be performed using commonly known methods. For example, when the host cell is prokaryotic, methods such as the CaCl2 method or electroporation may be used, and when the host cell is eukaryotic, methods such as microinjection, calcium phosphate precipitation, electroporation, liposome-mediated transfection, or gene bombardment may be used, but with no limitations thereto.

The method for selecting the transformed (recombinant vector-introduced) host cells can be easily performed using well-known methods in the art based on the phenotype expressed by a selectable marker. For example, if the selectable marker is a gene conferring resistance to a specific antibiotic, recombinant cells into which the recombinant vector has been introduced can be easily selected by culturing in a medium containing the antibiotic.

### Medical Uses

The anti-TIGIT antibody or antigen-binding fragment thereof included in the fusion protein provided herein has the ability to deliver IL-10 to effector immune cells to suppress immune cell activity (e.g., suppression of effector T cell function, promotion of Treg activity, reduced secretion of inflammatory cytokine, etc.). Additionally, IL-10 has high binding affinity to its receptor (IL-10 receptor) and low immunogenicity, resulting in immunosuppressant effect such as decreased cytokine (inflammatory cytokine) secretion when administered in vivo, while maintaining safety by minimizing the risk of being recognized as an immunogen. IL-10 also exhibits excellent autoimmunity and anti-inflammatory effects (e.g., inhibition of pro-inflammatory cytokine production and/or secretion, inhibition of effector immune cell proliferation, etc.). Therefore, the fusion peptide has the advantages of the anti-TIGIT antibody or its antigen-binding fragment and IL-10, and can be effectively applied for immune suppression, prevention and/or treatment of immune-related diseases, particularly for of autoimmune and/or inflammatory diseases.

An embodiment provides an immunosuppressant agent or a pharmaceutical composition for immunosuppression, comprising, as an active ingredient, at least one selected from the group consisting of the antibody or antigen-binding fragment thereof, or the fusion protein, a nucleic acid molecule encoding the antibody or antigen-binding fragment thereof or the fusion protein, a recombinant vector carrying the nucleic acid molecule, and a recombinant cell anchoring the nucleic acid molecule or recombinant vector.

Another embodiment provides a pharmaceutical composition for the prevention and/or treatment of immune-related diseases, the composition including, as an active ingredient, at least one selected from the group consisting of the antibody or antigen-binding fragment thereof, or the fusion protein, a nucleic acid molecule encoding the antibody or antigen-binding fragment thereof, or the fusion protein, a recombinant vector carrying the nucleic acid molecule, and a recombinant cell anchoring the nucleic acid molecule or recombinant vector. The above immune-related diseases may be selected from a group consisting of autoimmune diseases, inflammation, and inflammatory diseases.

Other embodiment provides a pharmaceutical composition (or anti-autoimmune agent and/or anti-inflammatory agent) for the prevention and/or treatment of autoimmunity (or autoimmune disease) and/or inflammation (or inflammatory disease) comprising, as an active ingredient, one or more selected from the group consisting of the above-mentioned antibody or antigen binding fragment, the above-mentioned fusion protein, the above-mentioned nucleic acid molecule encrypting the above-mentioned antibody or antigen binding fragment or fusion protein, the above-mentioned recombinant vector comprising the above-mentioned nucleic acid molecule, and the above-mentioned recombinant cell comprising the above-mentioned nucleic acid molecule or recombinant vector.

Another embodiment provides a method of suppressing immunity, the method comprising a step of administering a pharmaceutically effective amount of at least one selected from the group consisting of the antibody or antigen binding fragment or the fusion protein, a nucleic acid molecule encoding the fusion protein, a recombinant vector carrying the nucleic acid molecule, and a recombinant cell anchoring the nucleic acid molecule or recombinant vector, to a subject in need of immune suppression. The immune suppression method may further include the step of identifying the subject in need of immune suppression prior to the administration step.

Another embodiment provides a method of preventing and/or treating an immune-related disease, the method comprising the step of administering a pharmaceutically effective amount of at least one selected from the group consisting of the antibody or antigen binding fragment or the fusion protein, a nucleic acid molecule encoding the fusion protein, a recombinant vector carrying the nucleic acid molecule, and a recombinant cell anchoring the nucleic acid molecule or recombinant vector, to a subject in need of preventing and/or treating the immune-related disease. The method of preventing and/or treating immune-related diseases may further include a step of identifying the subject in need of preventing and/or treating the immune-related disease prior to the administration step.

Other embodiment provides a method for the prevention and/or treatment of autoimmunity (or autoimmune disease) and/or inflammation (or inflammatory disease) comprises the step of administering one or more pharmaceutically effective amounts selected from the group consisting of the antibody or antigen binding fragment, the fusion protein, a nucleic acid molecule encrypting the antibody or antigen binding fragment or fusion protein, a recombinant vector comprising the nucleic acid molecule, and a recombinant cell comprising the nucleic acid molecule or recombinant vector to a subject requiring prevention and/or treatment of autoimmunity (or autoimmune disease) and/or inflammation (or inflammatory disease). The method for the prevention and/or treatment of cancer may further include, prior to the step of administering, the step of identifying a subject requiring prevention and/or treatment of autoimmunity (or autoimmune disease) and/or inflammation (or inflammatory disease).

Other embodiment provides a use for the prevention and/or treatment of immunosuppression, immune-related diseases, and/or the prevention and/or treatment of autoimmunity (or autoimmune diseases) and/or inflammation (or inflammatory diseases), or a use for the preparation of a pharmaceutical composition for the prevention and/or treatment of immunosuppression, immune-related diseases, and/or the prevention and/or treatment of autoimmunity (or autoimmune diseases) and/or inflammation (or inflammatory diseases), of at least one selected from the group consisting of the antibody or antigen binding fragment, the fusion protein, a nucleic acid molecule encrypting the antibody or antigen binding fragment or the fusion protein, a recombinant vector comprising the nucleic acid molecule, and a recombinant cell comprising the nucleic acid molecule or the recombinant vector.

As used herein, the term "immune suppression" refers to the reduction of an initial immune response to an antigen or the attenuation of a pre-existing immune response, and may be used interchangeably with terms such as "immunosuppression" or "immune inactivation."

As used herein, the term "immune-related disease" is intended to encompass all diseases caused by immune system disorders and/or insufficient activation, including, but not limited to autoimmune diseases, inflammatory diseases and the like. The inflammation may be inflammation (e.g., chronic or acute inflammation) and may include, but is not limited to, inflammation caused by infection (e.g., local inflammation, systemic inflammation, etc.).

The autoimmune disease refers to or autoimmunity, or any symptom and/or disease caused by autoimmunity, and includes, but is not limited to, rheumatoid arthritis, type 1 diabetes, Crohn's disease, ulcerative colitis, Behcet's syndrome, lupus, and Sjögren's syndrome, and may be one or more selected from the group consisting of myasthenia gravis (e.g., myasthenia gravis), scleroderma, hypothyroidism, hyperthyroidism, psoriasis, vitiligo, multiple sclerosis, autoimmune hepatitis, autoimmune nephritis, autoimmune pancreatitis, autoimmune encephalitis, cytokine storm, etc., but is not limited thereto.

The inflammatory diseases refer to inflammation (e.g., chronic or acute inflammation) or diseases caused by inflammation, such as cardiac inflammation (e.g., coronary artery disease, angina pectoris, myocardial infarction, pericarditis, myocarditis, etc.), vascular inflammation (e.g., a collective term for atherosclerosis, arteriosclerosis, myocarditis, etc.), atherosclerosis, vasculitis, disseminated intravascular coagulation (DIC), immune thrombocytopenia (ITP), thrombotic thrombocytopenia (TTP), anemia), the inflammation shown in Fig. above (e.g., acute nasopharyngitis, allergic rhinitis, sinusitis, pharyngitis, tonsillitis, laryngitis), and Fig. below. and/or lung inflammation (e.g., bronchitis, bronchiectasis, asthma, chronic obstructive pulmonary disease (COPD), pneumonia, interstitial lung disease, tuberculosis), upper gastrointestinal inflammation (e.g., gastritis, esophagitis), lower gastrointestinal inflammation (e.g., small intestine disease, ulcerative colitis, Crohn's disease, celiac disease, diverticulitis, irritable bowel syndrome, appendicitis, fistula, etc.), liver, gallbladder Fig. and/or pancreatic inflammation (e.g., hepatitis, fatty liver, cholangitis, cholecystitis, pancreatitis, type 1 diabetes, etc.), renal (upper urinary tract) inflammation (e.g., pyelonephritis, glomerulonephritis, urinary tract infection), lower urinary tract inflammation (e.g., ureteritis, urethritis, cystitis, prostatitis/chronic pelvic pain syndrome, etc.), thyroid and/or parathyroid inflammation (e.g., thyroiditis, parathyroiditis, etc.), adrenal inflammation (e.g., adrenalitis, etc.), reproductive system inflammation (e.g., pelvic inflammatory disease, oophoritis, orchitis, epididymitis, etc.), bone and/or joint inflammation (e.g., osteoarthritis, rheumatoid arthritis, osteomyelitis, synovitis, etc.), skin inflammation (e.g., skin: soft tissue infection, tinea versicolor, athlete's foot, acne, etc.), muscle inflammation (e.g., myositis, etc.), brain inflammation (e.g., encephalitis, major depressive disorder, etc.), nerve inflammation (e.g., neuritis of various parts of the body such as the eyes and ears, complex regional pain syndrome, It may include one or more selected from the group consisting of Guillain-Barré syndrome, etc.), eye inflammation (e.g., blepharitis, uveitis, conjunctivitis, etc.), ear inflammation (otitis media, mastoiditis, etc.), oral inflammation (stomatitis, periodontitis, gingivitis, etc.), systemic inflammation (systemic inflammatory response syndrome (e.g., sepsis, etc.), metabolic syndrome-related diseases, etc.), peritonitis, reperfusion injury, transplant rejection, hypersensitivity reactions, etc., but is not limited thereto. The preventive and/or therapeutic effects of the autoimmune disease and/or inflammatory disease include, but are not limited to, suppressing the occurrence and/or increase of inflammation, as well as suppressing the exacerbation of autoimmunity and/or inflammation caused by migration, invasion, transmission, etc.

Systemic inflammation caused by the infection may be selected from any infectious disease that can be treated, alleviated, and/or prevented through immunosuppression as described above, including the inhibition of function (e.g., inactivation) and/or reduction of proliferation of immune cells (NK cells, effector T cells, cytotoxic T cells, CD3+ T cells, CD4+ T cells, CD8+ T cells, etc.), enhancement and/or stabilization of regulatory T cells (Treg), and reduction of production and/or secretion of inflammatory proteins (e.g., cytokines (TNF-alpha, IFN-gamma, etc.). For example, the infectious disease may be an infection by one or more pathogens selected from the group consisting of viruses (e.g., influenza viruses, coronaviruses (e.g., severe acute respiratory syndrome (SARS) viruses (SARS-CoV, SARS-CoV-2, etc.), Middle East respiratory syndrome (MERS) viruses (MERS-CoV), etc.), bacteria, fungi, parasites, etc.) or a disease (infectious disease) caused by such infection.

The subject to which the fusion protein and/or pharmaceutical composition provided herein may be administered may be any animal or cell. For example, the subject may be an animal selected from mammals including primates such as humans and monkeys, and rodents such as rats and mice, or cells, tissues, body fluids (e.g., serum), or cultures derived (isolated) from the animals, for example, human, or isolated cells, tissues, or body fluids (e.g., serum) derived from humans.

The pharmaceutical composition may additionally include a pharmaceutically acceptable carrier in addition to the fusion protein, nucleic acid molecule, vector, and/or cell as the active ingredient. The carrier may be any one commonly used for formulating protein drugs and may be selected from the group consisting of lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methylcellulose, methylparaben, propylparaben, talc, magnesium stearate, and mineral oil, but with no limitations thereto. The pharmaceutical composition may further include at least one selected from the group consisting of diluents, excipients, lubricants, wetting agents, sweeteners, flavoring agents, emulsifiers, suspending agents, and preservatives commonly used in the preparation of pharmaceutical compositions.

The content of the fusion protein, nucleic acid molecule, vector, and/or cell as the active ingredient in the pharmaceutical composition may range from 0.00001 to 99 wt%, 0.0001 to 99 wt%, 0.001 to 99 wt%, 0.01 to 99 wt%, 0.1 to 99 wt%, 1 to 99 wt%, 10 to 99 wt%, 15 to 99 wt%, 20 to 99 wt%, 25 to 99 wt%, 30 to 99 wt%, 35 to 99 wt%, 40 to 99 wt%, 45 to 99 wt%, or 50 to 99 wt%, based on the total weight of the composition, but is not limited thereto.

The administration of the fusion protein, nucleic acid molecule, vector, cells, and/or pharmaceutical composition may be via oral or non-oral routes. Non-oral administration may include intravenous injection, subcutaneous injection, intramuscular injection, intraperitoneal injection, intradermal injection, topical application, nasal administration, pulmonary administration, and rectal administration. For oral administration, since proteins or peptides are digested, oral compositions should be formulated to protect the active agent from degradation in the stomach.

Additionally, the fusion protein, nucleic acid molecule, vector, cell, and/or pharmaceutical composition may be formulated as a solution, suspension, syrup, or emulsion in an oil or aqueous medium, or as an extract, powder, granule, tablet, capsule, or injection, and may further include dispersants or stabilizers for formulation.

The content of the active ingredient in the pharmaceutical composition may vary depending on the method of formulation, route of administration, age, weight, sex, disease state of the patient, diet, time of administration, dosing interval, route of administration, rate of excretion, and response sensitivity. The daily dose of the pharmaceutical composition, based on the active ingredient (fusion protein, nucleic acid molecule, vector, cell), may range from 0.00001 to 1000 mg/kg, 0.00001 to 500 mg/kg, 0.00001 to 100 mg/kg, 0.00001 to 50 mg/kg, 0.0001 to 1000 mg/kg, 0.0001 to 500 mg/kg, 0.0001 to 100 mg/kg, 0.0001 to 50 mg/kg, 0.001 to 1000 mg/kg, 0.001 to 500 mg/kg, 0.001 to 100 mg/kg, 0.001 to 50 mg/kg, 0.01 to 1000 mg/kg, 0.01 to 500 mg/kg, 0.01 to 100 mg/kg, 0.01 to 50 mg/kg, 0.1 to 1000 mg/kg, 0.1 to 500 mg/kg, 0.1 to 100 mg/kg, or 0.1 to 50 mg/kg, but is not limited thereto. The daily dose may be formulated as a single unit dose or divided appropriately, or manufactured as a multi-dose container. As used herein, the term "pharmaceutically effective amount" refers to an amount of the active ingredient that exhibits the desired pharmaceutical activity, which may fall within the mentioned dosage ranges.

Another embodiment provides a health functional food for immune suppression, which includes, as an active ingredient, at least one selected from the group consisting of the fusion protein, a nucleic acid molecule encoding the antibody or antibody-binding fragment or the fusion protein, a recombinant vector carrying the nucleic acid molecule, and a recombinant cell anchoring the nucleic acid molecule or recombinant vector.

Another embodiment provides a health functional food for the prevention and/or improvement of immune-related diseases, the food including, as an active ingredient, at least one selected from the group consisting of the fusion protein, a nucleic acid molecule encoding the fusion protein, a recombinant vector carrying the nucleic acid molecule, and a recombinant cell anchoring the nucleic acid molecule or recombinant vector.

The term "health functional food", as used herein, refers to a food product manufactured (processed) using nutrients that are easily deficient in daily diet, or raw materials or ingredients with useful functions for the human body (hereinafter referred to as "functional raw materials"), and is intended to encompass all foods that are beneficial for maintaining health or preventing and/or alleviating certain diseases or symptoms. No particular limitations are imparted to the final product. For example, the health functional food may be selected from the group consisting of various foods, beverages, and food additives, but with no limitations thereto.

The food composition may include acceptable food additives, and may further include appropriate carriers, fillers, and diluents commonly used in the production of health functional foods.

When used as a food additive, the food composition may be added directly or used in conjunction with other foods or food ingredients, and may be appropriately used by conventional methods.

The content of the active ingredient in the food composition may be determined appropriately depending on the form of the food, the desired effect, and the purpose, and may range from 0.00001 to 99 wt%, 0.0001 to 99 wt%, 0.001 to 99 wt%, 0.01 to 99 wt%, 0.1 to 99 wt%, 1 to 99 wt%, or 10 to 99 wt% of the total food weight, but is not limited thereto.

### [Advantageous Effects]

The fusion protein provided herein possesses both the advantages of the previously described anti-TIGIT antibody or its antigen-binding fragment and IL-10, and can be usefully applied to prevention and/or treatment of immunosuppression, or immune-related diseases.

### [Brief Description of the Drawings]

Fig. 1 is a graph showing the results of measuring the degree of binding of anti-TIGIT antibody MMB0 and fusion protein (MMB5) provided according to an embodiment to human TIGIT by ELISA according to an embodiment.
Fig. 2 is a graph showing the results of measuring the degree of binding of anti-TIGIT antibody (MMB0) and fusion protein (MMB5) to IL-10Ra by ELISA.
Figs. 3a to 3c are graphs showing the results of measuring the binding affinity of fusion proteins provided according to an embodiment to TIGIT-expressing effector T cells and NK cells by FACS (3a: CD4+ T cell; 3b: CD8+ T cell; 3c: NK cell).
Fig. 4 is a graph showing the results of measuring by FACS whether the TIGIT/CD155 interaction of fusion proteins provided according to an embodiment is blocked.
Fig. 5a and 5b are graphs showing the results of confirming the STAT3 signaling levels of fusion proteins provided according to embodiment through STAT3 phosphorylation (5a: CD4+ T cell; 5b: CD8+ T cell).
Fig. 6 is a graph showing the transporting effect of IL-10 of the fusion protein to immune cells (effector T cells, NK cells) provided according to an embodiment.
Fig. 7 is a graph showing the inhibitory effect of fusion proteins on the production of inflammatory cytokines (TNF-α, Granzyme B) provided according to an embodiment.
Figs. 8a to 8c are graphs showing the inhibitory effect of fusion proteins on T cell proliferation according to an embodiment (8a: CD4+ T cell, 8b: CD8+ T cell, 8c: NK cell).
Figs. 9a to 9c are graphs showing the inhibitory effect of fusion proteins provided according to an embodiment on the expression of inflammatory cytokines (TNF-α) in PBMCs derived from patients with autoimmune diseases.
Fig. 10 is a graph showing the anti-inflammatory effect of the fusion protein provided according to an embodiment as a result of measuring the DAI score (Disease activity index score) in a DSS (Dextran sulfate sodium)-induced colitis model.

### [Mode for Invention]

Hereinafter, the present invention is to be described more specifically through examples, but this is merely illustrative and not intended to limit the scope of the present invention. It is obvious to those skilled in the art that the examples described below can be modified without departing from the essential essence of the invention.

### Example 1: Production of a Monoclonal Anti-TIGIT Antibody

### 1.1. Production of Mouse Monoclonal Anti-TIGIT Antibody

Four BALB/c mice were immunized by cross-injecting His-tagged human TIGIT Protein (R&D systems, UniProtKB/SwissProt Q495A1-1) at four times consecutively over 14 days. Lymphocytes were collected from the four mice, purified, and then fused with SP2/0 myeloma cells. The fused cells were proliferated in HAT selective single-step cloning media, and the resulting 2 x 920 hybridoma clones were selected by ClonePix2 (Molecular Devices), transferred to 96-well plates, and cultured.

Using indirect ELISA, the supernatant of hybridoma tissue cultures effective against recombinant human TIGIT (His) (R&D systems) was screened. For positive clones, Ig secretion and specificity were tested by additionally performing indirect ELISA on screening antigen (recombinant human TIGIT Fc chimeric protein (R&D systems)) and cells. The CHO-K1 (ATCC, CCL-61) cell line was used as a negative control.

The ELISA was performed under the following conditions;
- ELISA plates were coated at 4°C with a coating solution comprising 0.1 µg/well recombinant human TIGIT (rhTIGIT) protein at a concentration of 100 µL/well in Carbonate Coating Buffer (pH 9.6) at room temperature.
- Blocking at room temperature for 1 hour with 3% skim milk powder contained in PBS
- Treatment with secondary antibody 1:10000 Goat anti-mouse IgG/M(H+L)-HRP (100uL/well) contained in PBS-Tween for 1 hour (37°C, w/shaking).
- All washing steps are performed with PBS-Tween for 3 minutes.
- Adding TMB substrate at a concentration of 50 uL/well, leaving under dark conditions (5~10 mins), and stopping with an equal amount of 1 M HCl.

IgM-expressing clones were isolated and removed by isotyping, and IgG-expressing clones were collected.

The collected positive clones were subcloned to identify stable expression clones. The culture supernatant containing the selected clones was eluted by flowing it through a Protein A column, and the buffer was replaced with PBS. The purified antibodies were tested by performing an indirect ELISA against the screening antigen (Recombinant Human TIGIT (T103) Fc Chimera Protein; R&D systems) and the negative control antigen (His peptide).

Among the collected positive clones, the MMB0 clone (mouse backbone (mIgG1)-based mouse antibody) was selected as the primary antibody.

The CDR and variable regions of the antibodies are listed in Table 3 below.

**[Table 3]**

| | Amino acid sequence | SEQ ID NO |
|---|---|---|
| CDR-H1 | SDYAWN | 1 |
| CDR-H2 | YITYSGSTSYNPSLKS | 2 |
| CDR-H3 | RQVGLGFAY | 3 |
| CDR-L1 | KASQDVSNTVA | 4 |
| CDR-L2 | SASYRYT | 5 |
| CDR-L3 | QQHYSTPWT | 6 |
| Heavy chain variable region (VH0) | DVQLQESGPGLVKPSQSLSLTCTVTGYSITSDYAWN WIRQFPGNKLEWMAYITYSGSTSYNPSLKSRISITR DTSKNQFFLQLNSVTIEDTATYYCASRQVGLGFAY WGQGTLVTVSA | 29 |
| Light chain variable region (VK0) | DIVMTQSHKFMSTSVGDRVSITCKASQDVSNTVAW YQQRPGQSPKPLIYSASYRYTGVPDRFTGSGSGTDF TFTISSVQAEDLAVYYCQQHYSTPWTFGGGTKLEIK | 35 |

### 1.2. Humanization of Mouse Anti-TIGIT Antibody

To identify amino acid residues critical to antibody structure and binding, protein structure models of monoclonal antibody variable regions (mAb V regions) were analyzed. Using this information in conjunction with in silico designs of human antibody structures, a large number of preliminary sequence fragments suitable for the production of humanized variants of MMB0 were selected, and peptide binding to human MHC class II alleles was analyzed. Where possible, sequence fragments identified as significant non-human germline binders for human MHC class II were discarded. This reduced the set of fragments, and these combinations were re-analyzed using the aforementioned method to identify that the junctions between fragments did not contain potential T cell epitopes. The selected sequence fragments were assembled to generate a complete variable region (V region) that either did not contain or had reduced significant T cell epitopes, thereby designing a human variable region that evades potential T cell epitope recognition (deimmunization).

Six heavy chain variable regions (VH1, VH6, VH7, VH5, VH8, VH9) and seven light chain variable regions (Vk1, Vk2, Vk7, Vk11, Vk5, Vk10, Vk14) sequences for preparing humanized antibodies were selected for use in gene synthesis and expression in mammalian cells.

The sequences of the six heavy chain variable regions and seven light chain variable regions for the preparation of the humanized antibody, as well as the heavy chain variable region (VH0) and light chain variable region (Vk0) of the mouse antibody MMB0, are summarized in Table 4 below.

**[Table 4]**

| Heavy chain variable region | Amino acid sequence (N→C) | SEQ ID NO |
|---|---|---|
| VH0 | DVQLQESGPGLVKPSQSLSLTCTVTGYSITSDYAW NWIRQFPGNKLEWMAYITYSGSTSYNPSLKSRISIT RDTSKNQFFLQLNSVTIEDTATYYCASRQVGLGFA YWGQGTLVTVSA | 29 |
| VH1 | DVQLQESGPGLVKPSQTLSLTCTVTGYSITSDYAW NWIRQPPGKGLEWMAYITYSGSTSYNPSLKSRITIS RDTSKNQFSLKLSSVTIEDTATYYCASRQVGLGFA YWGQGTLVTVSS | 30 |
| VH6 | DVQLQESGPGLVKPSQTLSLTCTVTGYSITSDYAW NWIRQPPGKGLEWMAYITYSGSTSYNPSLKSRITIS RDTSKNQFFLKLSSVTIEDTATYYCASRQVGLGFA YWGQGTLVTVSS | 31 |
| VH7 | DVQLQESGPGLVKPSQTLSLTCTVTGYSITSDYAW NWIRQFPGNKLEWMAYITYSGSTSYNPSLKSRITIS RDTSKNQFSLKLSSVTIEDTATYYCASRQVGLGFA YWGQGTLVTVSS | 32 |
| VH5 | QVQLQESGPGLVKPSQTLSLTCTVTGYSITSDYAW NWIRQPPGKGLEWMAYITYSGSTSYNPSLKSRVTI SVDTSKNQFSLKLSSVTAADTAVYYCASRQVGLG FAYWGQGTLVTVSS | 33 |
| VH8 | QVQLQESGPGLVKPSQTLSLTCTVTGYSITSDYAW NWIRQPPGKGLEWMAYITYSGSTSYNPSLKSRVTI SVDTSKNQFFLKLSSVTAADTAVYYCASRQVGLG FAYWGQGTLVTVSS | 34 |
| VH9 | QVQLQESGPGLVKPSQTLSLTCTVTGYSITSDYAW NWIRQFPGNKLEWMAYITYSGSTSYNPSLKSRVTI SVDTSKNQFSLKLSSVTAADTAVYYCASRQVGLG FAYWGQGTLVTVSS | 7 |

| Light chain variable region | Amino acid sequence (N→C) | SEQ ID NO |
|---|---|---|
| Vk0 | DIVMTQSHKFMSTSVGDRVSITCKASQDVSNTVA WYQQRPGQSPKPLIYSASYRYTGVPDRFTGSGSG TDFTFTISSVQAEDLAVYYCQQHYSTPWTFGGGT KLEIK | 35 |
| Vk1 | DIVMTQSHSFLSASVGDRVSITCKASQDVSNTVAW YQQKPGQPPKPLIYSASYRYTGVPDRFTGSGSGTD FTLTISSLQAEDVAVYYCQQHYSTPWTFGGGTKVE IK | 36 |
| Vk2 | DIVMTQSPSSLSASVGDRVSITCKASQDVSNTVAW YQQKPGQPPKPLIYSASYRYTGVPDRFTGSGSGTD FTLTISSLQAEDVAVYYCQQHYSTPWTFGGGTKVE IK | 37 |
| Vk7 | DIVMTQSPSSLSASVGDRVSITCKASQDVSNTVAW YQQKPGQAPRPLIYSASYRYTGVPDRFTGSGSGT DFTLTISSLQAEDVAVYYCQQHYSTPWTFGGGTK VEIK | 38 |
| Vk11 | DIVMTQSHSSLSASVGDRVSITCKASQDVSNTVAW YQQKPGQPPKPLIYSASYRYTGVPDRFTGSGSGTD FTLTISSLQAEDVAVYYCQQHYSTPWTFGGGTKVE IK | 8 |
| Vk5 | DIQMTQSPSSLSASVGDRVSITCKASQDVSNTVAW YQQKPGQPPKPLIYSASYRYTGVPDRFSGSGSGTD FTLTISSLQPEDVATYYCQQHYSTPWTFGGGTKVE IK | 39 |
| Vk10 | DIQMTQSPSSLSASVGDRVSITCKASQDVSNTVAW YQQKPGQAPRPLIYSASYRYTGVPDRFSGSGSGTD FTLTISSLQPEDVATYYCQQHYSTPWTFGGGTKVE IK | 40 |
| Vk14 | DIQMTQSPSFLSASVGDRVSITCKASQDVSNTVAW YQQKPGQPPKPLIYSASYRYTGVPDRFSGSGSGTD FTLTISSLQPEDVATYYCQQHYSTPWTFGGGTKVE IK | 41 |

In addition, the human IgG-derived constant regions used in the preparation of the humanized antibody are summarized in Table 5 below:

**[Table 5]**

| | Amino acid sequence (N→C) | SEQ ID NO |
|---|---|---|
| Heavy chain constant region | ASTKGPSVFPLAPCSRSTSESTAALGCLVKDYFPEPVTV SWNSGALTSGVHTFPAVLQSSGLYSLSSVVTVPSSSLGT KTYTCNVDHKPSNTKVDKRVESKYGPPCPPCPAPEFLG GPSVFLFPPKPKDTLMISRTPEVTCVVVDVSQEDPEVQF NWYVDGVEVHNAKTKPREEQFNSTYRVVSVLTVLHQ DWLNGKEYKCKVSNKGLPSSIEKTISKAKGQPREPQVY TLPPSQEEMTKNQVSLTCLVKGFYPSDIAVEWESNGQP ENNYKTTPPVLDSDGSFFLYSRLTVDKSRWQEGNVFSC SVMHEALHNHYTQKSLSLSLGK | 10 |
| Light chain constant region | | 12 |

Based on the variable region sequences listed in Tables 4 and 5 above, various combinations of humanized antibodies were constructed using the sequences of six heavy chain variable regions (VH1, VH6, VH7, VH5, VH8, VH9), seven light chain variable regions (Vk1, Vk2, Vk7, Vk11, Vk5, Vk10, Vk14), and the human constant region sequence of Table 5, and chimeric antibodies were constructed using the VH0/VK0 combination, which is the variable region of a mouse antibody, and the human constant region of Table 5. Among these, a humanized antibody constructed using the combination of VH9 (SEQ ID NO: 7) and Vk11 (SEQ ID NO:8) (hereinafter referred to as "MMB0" or "MMB0_VH9/Vk11") was selected and used in the following working examples.

### Example 2. Preparation of [Anti-TIGIT Monoclonal Antibody]-[Wild-type IL-10] Fusion Protein

A fusion protein was designed to have the structure shown in Table 6 below by fusing the humanized anti-TIGIT antibody prepared in Example 1.2 and IL-10 with or without a linker.

**[Table 6]**

| Light and heavy chain TIGIT mAb-wild type IL-10 fusion proteins | | |
|---|---|---|
| Fusion protein name | Structure | Linker |
| MMB0-IL-10 monomer (Fusion protein of TIGIT mAbMMB0 with wild type IL-10 at LC linking) | TIGIT mAb-(LC-(EAAAK)3-(IL-10)2 | Wild type IL-10 fused to LC C-terminus (LC variant) with rigid linker |
| | TIGIT mAb-(LC-(GGGGS)3-(IL-10)2 | Wild type IL-10 fused to LC C-terminus (LC variant) with flexible linker |
| MMB0-IL-10(monomer)-Fc (Fusion protein of TIGIT mAbMMB0 with wild type IL-10 at FC linking) | TIGIT mAb-Fc-((EAAAK)3-(IL-10)2 | Wild type IL-10 fused to Fc C-terminus (Fc variant) with rigid linker |
| | TIGIT mAb-Fc-((GGGGS)3-(IL-10)2 | Wild type IL-10 fused to Fc C-terminus (Fc variant) with flexible linker |
| | TIGIT mAb-Fc-(IL-10)2 | Wild type IL-10 fused to Fc C-terminus (Fc variant) without linker |
| MMB0-IL-10 dimer (Fusion protein of TIGIT mAbMMB0 with wild type IL-10 dimer at LC linking) | TIGIT mAb-(LC-(EAAAK)3-(IL-10 dimer)2 | Wild type IL-10 dimer fused to LC C-terminus (LC variant) with rigid linker |
| | TIGIT mAb-(LC-(GGGGS)3-(IL-10 dimer)2 | Wild type IL-10 dimer fused to LC C-terminus (LC variant) with flexible linker |
| MMB0-IL-10(dimer)-Fc (Fusion protein of TIGIT mAbMMB0 with wild type IL-10 dimer at FC linking) | TIGIT mAb-Fc-((EAAAK)3-(IL-10 dimer)2 | Wild type IL-10 dimer fused to Fc C-terminus (Fc variant) with rigid linker |
| | TIGIT mAb-Fc-((GGGGS)3-(IL-10 dimer)2 | Wild type IL-10 dimer fused to Fc C-terminus (Fc variant) with flexible linker |
| | TIGIT mAb-Fc-(IL-10 dimer)2 | Wild type IL-10 dimer fused to Fc C-terminus (Fc variant) without linker |

(In Table 6 above,
Anti-TIGIT monoclonal antibody(TIGIT mAb): MMB0_VH9/Vk11 (hereinafter, 'MMB0');
IL-10: wild type IL-10;
Two linkers or no linker: LC variants (fused to LC(light chain) C-terminus with rigid and flexible linker) & Fc variants (ΔK478) (fused to Fc C-terminus without linker and rigid and flexible linker))

A gene encoding the TIGIT mAb (see Table 7) was synthesized and cloned into a modified pcDNA3.4 vector (Biointron) including a 'linker-wild type IL-10 element'. CHO-K1 cells (ATCC) were seeded onto a plate and cultured to a density of 70-90%, and then, the gene DNA was transfected into the CHO-K1 cells by electrophoresis. Subsequently, the cells were cultured at 37°C to induce transient expression. The culture supernatant was purified by Protein A affinity chromatography and preparative SEC. The produced protein was buffered with PBS, quantified by OD280 nm measurement, and analyzed by SDS-PAGE.

**[Table 7]**

| Sequences of anti-TIGIT mAb and fusion protein MMB0-IL-10 | | |
|---|---|---|
| Components | Amino acid sequence (N→C) or nucleic acid sequence (5'→3') | SEQ ID NO |
| Anti-TIGIT heavy chain variable region | QVQLQESGPGLVKPSQTLSLTCTVTGYSITSDYAWN WIRQFPGNKLEWMAYITYSGSTSYNPSLKSRVTISVD TSKNQFSLKLSSVTAADTAVYYCASRQVGLGFAYWG QGTLVTVSS | 7 |
| Anti-TIGIT heavy chain constant region | ASTKGPSVFPLAPCSRSTSESTAALGCLVKDYFPEPV TVSWNSGALTSGVHTFPAVLQSSGLYSLSSVVTVPS SSLGTKTYTCNVDHKPSNTKVDKRVESKYGPPCPPC PAPEFLGGPSVFLFPPKPKDTLMISRTPEVTCVVVDV SQEDPEVQFNWYVDGVEVHNAKTKPREEQFNSTYR VVSVLTVLHQDWLNGKEYKCKVSNKGLPSSIEKTIS KAKGQPREPQVYTLPPSQEEMTKNQVSLTCLVKGF YPSDIAVEWESNGQPENNYKTTPPVLDSDGSFFLYS RLTVDKSRWQEGNVFSCSVMHEALHNHYTQKSLSL SLGK | 10 |
| Anti-TIGIT heavy chain | QVQLQESGPGLVKPSQTLSLTCTVTGYSITSDYAWN WIRQFPGNKLEWMAYITYSGSTSYNPSLKSRVTISV DTSKNQFSLKLSSVTAADTAVYYCASRQVGLGFAY WGQGTLVTVSSASTKGPSVFPLAPCSRSTSESTAALG CLVKDYFPEPVTVSWNSGALTSGVHTFPAVLQSSGL YSLSSVVTVPSSSLGTKTYTCNVDHKPSNTKVDKRV ESKYGPPCPPCPAPEFLGGPSVFLFPPKPKDTLMISRT PEVTCVVVDVSQEDPEVQFNWYVDGVEVHNAKTK PREEQFNSTYRVVSVLTVLHQDWLNGKEYKCKVSN KGLPSSIEKTISKAKGQPREPQVYTLPPSQEEMTKNQ VSLTCLVKGFYPSDIAVEWESNGQPENNYKTTPPVL DSDGSFFLYSRLTVDKSRWQEGNVFSCSVMHEALH NHYTQKSLSLSLGK | 11 |
| Anti-TIGIT Light Chain Variable Region | DIVMTQSHSSLSASVGDRVSITCKASQDVSNTVAWY QQKPGQPPKPLIYSASYRYTGVPDRFTGSGSGTDFTL TISSLQAEDVAVYYCQQHYSTPWTFGGGTKVEIK | 8 |
| Anti-TIGIT Light Chain Constant region | | 12 |
| Anti-TIGIT light chains | DIVMTQSHSSLSASVGDRVSITCKASQDVSNTVAWY QQKPGQPPKPLIYSASYRYTGVPDRFTGSGSGTDFTL TISSLQAEDVAVYYCQQHYSTPWTFGGGTKVEIKRT VAAPSVFIFPPSDEQLKSGTASVVCLLNNFYPREAKV QWKVDNALQSGNSQESVTEQDSKDSTYSLSSTLTLS KADYEKHKVYACEVTHQGLSSPVTKSFNRGEC | 13 |
| IL-10 Polypeptides | SPGQGTQSENSCTHFPGNLPNMLRDLRDAFSRVKTF FQMKDQLDNLLLKESLLEDFKGYLGCQALSEMIQFY LEEVMPQAENQDPDIKAHVNSLGENLKTLRLRLRRC HRFLPCENKSKAVEQVKNAFNKLQEKGIYKAMSEFD IFINYIEAYMTMKIRN | 9 |
| Linker (V-1) | EAAAKEAAAKEAAAK | 14 |
| Linker (V-2) | GGGGSGGGGSGGGGS | 15 |
| Linker (D-1) | GGGSGGG | 16 |

| **IL-10 monomer (two) is linked without a linker to the respective C terminus of the light chains (two) of the anti-TIGIT antibody MMB0** | | |
|---|---|---|
| Heavy chain strand (anti-TIGIT heavy chain (IgG1)) | QVQLQESGPGLVKPSQTLSLTCTVTGYSITSDYAWN WIRQFPGNKLEWMAYITYSGSTSYNPSLKSRVTISVD TSKNQFSLKLSSVTAADTAVYYCASRQVGLGFAYWG QGTLVTVSSASTKGPSVFPLAPCSRSTSESTAALGCL VKDYFPEPVTVSWNSGALTSGVHTFPAVLQSSGLYSL SSVVTVPSSSLGTKTYTCNVDHKPSNTKVDKRVESK YGPPCPPCPAPEFLGGPSVFLFPPKPKDTLMISRTPEV TCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL TCLVKGFYPSDIAVEWESNGQPENNYKTTPPVLDSD GSFFLYSRLTVDKSRWQEGNVFSCSVMHEALHNHYT QKSLSLSLGK | 11 |
| Light chain strand ([Anti-TIGIT light chain (kappa)]-[IL-10]) | DIVMTQSHSSLSASVGDRVSITCKASQDVSNTVAWY QQKPGQPPKPLIYSASYRYTGVPDRFTGSGSGTDFTL TISSLQAEDVAVYYCQQHYSTPWTFGGGTKVEIKRT VAAPSVFIFPPSDEQLKSGTASVVCLLNNFYPREAKV QWKVDNALQSGNSQESVTEQDSKDSTYSLSSTLTLS KADYEKHKVYACEVTHQGLSSPVTKSFNRGECSPG QGTQSENSCTHFPGNLPNMLRDLRDAFSRVKTFFQM KDQLDNLLLKESLLEDFKGYLGCQALSEMIQFYLEE VMPQAENQDPDIKAHVNSLGENLKTLRLRLRRCHR FLPCENKSKAVEQVKNAFNKLQEKGIYKAMSEFDIFI NYIEAYMTMKIRN | 17 |

| **IL-10 monomer (2) is linked via linker V-1 to the respective C terminus of the light chains (2) of the anti-TIGIT antibody MMB0** | | |
|---|---|---|
| Heavy chain strand (anti-TIGIT heavy chain (IgG1)) | QVQLQESGPGLVKPSQTLSLTCTVTGYSITSDYAWN WIRQFPGNKLEWMAYITYSGSTSYNPSLKSRVTISV DTSKNQFSLKLSSVTAADTAVYYCASRQVGLGFAY WGQGTLVTVSSASTKGPSVFPLAPCSRSTSESTAALG CLVKDYFPEPVTVSWNSGALTSGVHTFPAVLQSSGL YSLSSVVTVPSSSLGTKTYTCNVDHKPSNTKVDKRV ESKYGPPCPPCPAPEFLGGPSVFLFPPKPKDTLMISRT PEVTCVVVDVSQEDPEVQFNWYVDGVEVHNAKTK PREEQFNSTYRVVSVLTVLHQDWLNGKEYKCKVSN KGLPSSIEKTISKAKGQPREPQVYTLPPSQEEMTKNQ VSLTCLVKGFYPSDIAVEWESNGQPENNYKTTPPVL DSDGSFFLYSRLTVDKSRWQEGNVFSCSVMHEALH NHYTQKSLSLSLGK | 11 |
| Light chain strand ([Anti-TIGIT light chain (kappa)]-[Linker (V-1)]-[IL-10]) | DIVMTQSHSSLSASVGDRVSITCKASQDVSNTVAWY QQKPGQPPKPLIYSASYRYTGVPDRFTGSGSGTDFTL TISSLQAEDVAVYYCQQHYSTPWTFGGGTKVEIKRT VAAPSVFIFPPSDEQLKSGTASVVCLLNNFYPREAKV QWKVDNALQSGNSQESVTEQDSKDSTYSLSSTLTLS KADYEKHKVYACEVTHQGLSSPVTKSFNRGECEAA AKEAAAKEAAAKSPGQGTQSENSCTHFPGNLPNML RDLRDAFSRVKTFFQMKDQLDNLLLKESLLEDFKGY LGCQALSEMIQFYLEEVMPQAENQDPDIKAHVNSLG ENLKTLRLRLRRCHRFLPCENKSKAVEQVKNAFNKL QEKGIYKAMSEFDIFINYIEAYMTMKIRN | 18 |

| **IL-10 monomer (2) is linked via linker V-2 to each C-terminus of the light chain (2) of the anti-TIGIT antibody MMB0 (MMB2)** | | |
|---|---|---|
| Heavy chain strand (anti-TIGIT heavy chain (IgG1)) | QVQLQESGPGLVKPSQTLSLTCTVTGYSITSDYAWN WIRQFPGNKLEWMAYITYSGSTSYNPSLKSRVTISVD TSKNQFSLKLSSVTAADTAVYYCASRQVGLGFAYWG QGTLVTVSSASTKGPSVFPLAPCSRSTSESTAALGCL VKDYFPEPVTVSWNSGALTSGVHTFPAVLQSSGLYSL SSVVTVPSSSLGTKTYTCNVDHKPSNTKVDKRVESK YGPPCPPCPAPEFLGGPSVFLFPPKPKDTLMISRTPEV TCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL TCLVKGFYPSDIAVEWESNGQPENNYKTTPPVLDSD GSFFLYSRLTVDKSRWQEGNVFSCSVMHEALHNHYT QKSLSLSLGK | 11 |
| Light chain strand ([Anti-TIGIT light chain (kappa)]-[Linker (V-2)]-[IL-10]) | DIVMTQSHSSLSASVGDRVSITCKASQDVSNTVAWY QQKPGQPPKPLIYSASYRYTGVPDRFTGSGSGTDFTL TISSLQAEDVAVYYCQQHYSTPWTFGGGTKVEIKRT VAAPSVFIFPPSDEQLKSGTASVVCLLNNFYPREAKV QWKVDNALQSGNSQESVTEQDSKDSTYSLSSTLTLS KADYEKHKVYACEVTHQGLSSPVTKSFNRGECGGG GSGGGGSGGGGSSPGQGTQSENSCTHFPGNLPNMLR DLRDAFSRVKTFFQMKDQLDNLLLKESLLEDFKGYL GCQALSEMIQFYLEEVMPQAENQDPDIKAHVNSLGE NLKTLRLRLRRCHRFLPCENKSKAVEQVKNAFNKLQ EKGIYKAMSEFDIFINYIEAYMTMKIRN | 19 |

| **IL-10 monomer (2) is linked without a linker to each C-terminus of the heavy chain (2) of the anti-TIGIT antibody MMB0** | | |
|---|---|---|
| Heavy chain strand ([anti-TIGIT heavy chain (IgG1)]-[IL-10]) | QVQLQESGPGLVKPSQTLSLTCTVTGYSITSDYAWN WIRQFPGNKLEWMAYITYSGSTSYNPSLKSRVTISVD TSKNQFSLKLSSVTAADTAVYYCASRQVGLGFAYWG QGTLVTVSSASTKGPSVFPLAPCSRSTSESTAALGCL VKDYFPEPVTVSWNSGALTSGVHTFPAVLQSSGLYSL SSVVTVPSSSLGTKTYTCNVDHKPSNTKVDKRVESK YGPPCPPCPAPEFLGGPSVFLFPPKPKDTLMISRTPEV TCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL TCLVKGFYPSDIAVEWESNGQPENNYKTTPPVLDSD GSFFLYSRLTVDKSRWQEGNVFSCSVMHEALHNHYT QKSLSLSLGKSPGQGTQSENSCTHFPGNLPNMLRDL RDAFSRVKTFFQMKDQLDNLLLKESLLEDFKGYLGC QALSEMIQFYLEEVMPQAENQDPDIKAHVNSLGENL KTLRLRLRRCHRFLPCENKSKAVEQVKNAFNKLQEK GIYKAMSEFDIFINYIEAYMTMKIRN | 20 |
| Light chain strand (Anti-TIGIT light chain (kappa)) | DIVMTQSHSSLSASVGDRVSITCKASQDVSNTVAWY QQKPGQPPKPLIYSASYRYTGVPDRFTGSGSGTDFTL TISSLQAEDVAVYYCQQHYSTPWTFGGGTKVEIKRT VAAPSVFIFPPSDEQLKSGTASVVCLLNNFYPREAKV QWKVDNALQSGNSQESVTEQDSKDSTYSLSSTLTLS KADYEKHKVYACEVTHQGLSSPVTKSFNRGEC | 13 |

| **IL-10 monomer (2) is linked via linker V-1 to each C-terminus of the heavy chain (2) of the anti-TIGIT antibody MMB0** | | |
|---|---|---|
| Heavy chain strand ([anti-TIGIT heavy chain (IgG1)]-[linker (V-1)]-[IL-10]) | QVQLQESGPGLVKPSQTLSLTCTVTGYSITSDYAWN WIRQFPGNKLEWMAYITYSGSTSYNPSLKSRVTISVD TSKNQFSLKLSSVTAADTAVYYCASRQVGLGFAYWG QGTLVTVSSASTKGPSVFPLAPCSRSTSESTAALGCL VKDYFPEPVTVSWNSGALTSGVHTFPAVLQSSGLYSL SSVVTVPSSSLGTKTYTCNVDHKPSNTKVDKRVESK YGPPCPPCPAPEFLGGPSVFLFPPKPKDTLMISRTPEV TCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL TCLVKGFYPSDIAVEWESNGQPENNYKTTPPVLDSD GSFFLYSRLTVDKSRWQEGNVFSCSVMHEALHNHYT QKSLSLSLGKEAAAKEAAAKEAAAKSPGQGTQSEN SCTHFPGNLPNMLRDLRDAFSRVKTFFQMKDQLDN LLLKESLLEDFKGYLGCQALSEMIQFYLEEVMPQAE NQDPDIKAHVNSLGENLKTLRLRLRRCHRFLPCENK SKAVEQVKNAFNKLQEKGIYKAMSEFDIFINYIEAY MTMKIRN | 21 |
| Light chain strand (Anti-TIGIT light chain (kappa)) | DIVMTQSHSSLSASVGDRVSITCKASQDVSNTVAWY QQKPGQPPKPLIYSASYRYTGVPDRFTGSGSGTDFTL TISSLQAEDVAVYYCQQHYSTPWTFGGGTKVEIKRT VAAPSVFIFPPSDEQLKSGTASVVCLLNNFYPREAKV QWKVDNALQSGNSQESVTEQDSKDSTYSLSSTLTLS KADYEKHKVYACEVTHQGLSSPVTKSFNRGEC | 13 |

| **IL-10 monomer (2) is linked via linker V-2 to each C-terminus of the light chain (2) of the anti-TIGIT antibody MMB0 (MMB1)** | | |
|---|---|---|
| Heavy chain strand ([anti-TIGIT heavy chain (IgG1)]-[linker (V-2)]-[IL-10]) | QVQLQESGPGLVKPSQTLSLTCTVTGYSITSDYAWN WIRQFPGNKLEWMAYITYSGSTSYNPSLKSRVTISVD TSKNQFSLKLSSVTAADTAVYYCASRQVGLGFAYWG QGTLVTVSSASTKGPSVFPLAPCSRSTSESTAALGCL VKDYFPEPVTVSWNSGALTSGVHTFPAVLQSSGLYSL SSVVTVPSSSLGTKTYTCNVDHKPSNTKVDKRVESK YGPPCPPCPAPEFLGGPSVFLFPPKPKDTLMISRTPEV TCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL TCLVKGFYPSDIAVEWESNGQPENNYKTTPPVLDSD GSFFLYSRLTVDKSRWQEGNVFSCSVMHEALHNHYT QKSLSLSLGKGGGGSGGGGSGGGGSSPGQGTQSENS CTHFPGNLPNMLRDLRDAFSRVKTFFQMKDQLDNL LLKESLLEDFKGYLGCQALSEMIQFYLEEVMPQAEN QDPDIKAHVNSLGENLKTLRLRLRRCHRFLPCENKS KAVEQVKNAFNKLQEKGIYKAMSEFDIFINYIEAYM TMKIRN | 22 |
| Light chain strand (Anti-TIGIT light chain (kappa)) | DIVMTQSHSSLSASVGDRVSITCKASQDVSNTVAWY QQKPGQPPKPLIYSASYRYTGVPDRFTGSGSGTDFTL TISSLQAEDVAVYYCQQHYSTPWTFGGGTKVEIKRT VAAPSVFIFPPSDEQLKSGTASVVCLLNNFYPREAKV QWKVDNALQSGNSQESVTEQDSKDSTYSLSSTLTLS KADYEKHKVYACEVTHQGLSSPVTKSFNRGEC | 13 |

| **IL-10 dimer (2) is linked without a linker to the respective C terminus of the light chains (2) of the anti-TIGIT antibody MMB0** | | |
|---|---|---|
| Heavy chain strand (anti-TIGIT heavy chain (IgG1)) | QVQLQESGPGLVKPSQTLSLTCTVTGYSITSDYAWN WIRQFPGNKLEWMAYITYSGSTSYNPSLKSRVTISVD TSKNQFSLKLSSVTAADTAVYYCASRQVGLGFAYWG QGTLVTVSSASTKGPSVFPLAPCSRSTSESTAALGCL VKDYFPEPVTVSWNSGALTSGVHTFPAVLQSSGLYSL SSVVTVPSSSLGTKTYTCNVDHKPSNTKVDKRVESK YGPPCPPCPAPEFLGGPSVFLFPPKPKDTLMISRTPEV TCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL TCLVKGFYPSDIAVEWESNGQPENNYKTTPPVLDSD GSFFLYSRLTVDKSRWQEGNVFSCSVMHEALHNHYT QKSLSLSLGK | 11 |
| Light chain strand ([Anti-TIGIT light chain (kappa)]-[IL-10]-[D-1]-[IL-10]) | DIVMTQSHSSLSASVGDRVSITCKASQDVSNTVAWY QQKPGQPPKPLIYSASYRYTGVPDRFTGSGSGTDFTL TISSLQAEDVAVYYCQQHYSTPWTFGGGTKVEIKRT VAAPSVFIFPPSDEQLKSGTASVVCLLNNFYPREAKV QWKVDNALQSGNSQESVTEQDSKDSTYSLSSTLTLS KADYEKHKVYACEVTHQGLSSPVTKSFNRGECSPG QGTQSENSCTHFPGNLPNMLRDLRDAFSRVKTFFQM KDQLDNLLLKESLLEDFKGYLGCQALSEMIQFYLEE VMPQAENQDPDIKAHVNSLGENLKTLRLRLRRCHR FLPCENKSKAVEQVKNAFNKLQEKGIYKAMSEFDIFI NYIEAYMTMKIRNGGGSGGGSPGQGTQSENSCTHFP GNLPNMLRDLRDAFSRVKTFFQMKDQLDNLLLKES LLEDFKGYLGCQALSEMIQFYLEEVMPQAENQDPDI KAHVNSLGENLKTLRLRLRRCHRFLPCENKSKAVEQ VKNAFNKLQEKGIYKAMSEFDIFINYIEAYMTMKIR N | 23 |

| **IL-10 dimer (2) is linked via linker V-1 to the respective C terminus of the light chains (2) of the anti-TIGIT antibody MMB0 (MMB5)** | | |
|---|---|---|
| Heavy chain strand (anti-TIGIT heavy chain (IgG1)) | QVQLQESGPGLVKPSQTLSLTCTVTGYSITSDYAWN WIRQFPGNKLEWMAYITYSGSTSYNPSLKSRVTISV DTSKNQFSLKLSSVTAADTAVYYCASRQVGLGFAY WGQGTLVTVSSASTKGPSVFPLAPCSRSTSESTAALG CLVKDYFPEPVTVSWNSGALTSGVHTFPAVLQSSGL YSLSSVVTVPSSSLGTKTYTCNVDHKPSNTKVDKRV ESKYGPPCPPCPAPEFLGGPSVFLFPPKPKDTLMISRT PEVTCVVVDVSQEDPEVQFNWYVDGVEVHNAKTK PREEQFNSTYRVVSVLTVLHQDWLNGKEYKCKVSN KGLPSSIEKTISKAKGQPREPQVYTLPPSQEEMTKNQ VSLTCLVKGFYPSDIAVEWESNGQPENNYKTTPPVL DSDGSFFLYSRLTVDKSRWQEGNVFSCSVMHEALH NHYTQKSLSLSLGK | 11 |
| Light chain strand ([Anti-TIGIT light chain (kappa)]-[Linker (V-1)]-[IL-10]-[D-1]-[IL-10]) | DIVMTQSHSSLSASVGDRVSITCKASQDVSNTVAWY QQKPGQPPKPLIYSASYRYTGVPDRFTGSGSGTDFTL TISSLQAEDVAVYYCQQHYSTPWTFGGGTKVEIKRT VAAPSVFIFPPSDEQLKSGTASVVCLLNNFYPREAKV QWKVDNALQSGNSQESVTEQDSKDSTYSLSSTLTLS KADYEKHKVYACEVTHQGLSSPVTKSFNRGECEAA AKEAAAKEAAAKSPGQGTQSENSCTHFPGNLPNML RDLRDAFSRVKTFFQMKDQLDNLLLKESLLEDFKGY LGCQALSEMIQFYLEEVMPQAENQDPDIKAHVNSLG ENLKTLRLRLRRCHRFLPCENKSKAVEQVKNAFNKL QEKGIYKAMSEFDIFINYIEAYMTMKIRNGGGSGGGS PGQGTQSENSCTHFPGNLPNMLRDLRDAFSRVKTFF QMKDQLDNLLLKESLLEDFKGYLGCQALSEMIQFY LEEVMPQAENQDPDIKAHVNSLGENLKTLRLRLRRC HRFLPCENKSKAVEQVKNAFNKLQEKGIYKAMSEFD IFINYIEAYMTMKIRN | 24 |

| **IL-10 dimer (2) is linked via linker V-2 to each C-terminus of the light chain (2) of the anti-TIGIT antibody MMB0 (MMB6)** | | |
|---|---|---|
| Heavy chain strand (anti-TIGIT heavy chain (IgG1)) | QVQLQESGPGLVKPSQTLSLTCTVTGYSITSDYAWN WIRQFPGNKLEWMAYITYSGSTSYNPSLKSRVTISVD TSKNQFSLKLSSVTAADTAVYYCASRQVGLGFAYWG QGTLVTVSSASTKGPSVFPLAPCSRSTSESTAALGCL VKDYFPEPVTVSWNSGALTSGVHTFPAVLQSSGLYSL SSVVTVPSSSLGTKTYTCNVDHKPSNTKVDKRVESK YGPPCPPCPAPEFLGGPSVFLFPPKPKDTLMISRTPEV TCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL TCLVKGFYPSDIAVEWESNGQPENNYKTTPPVLDSD GSFFLYSRLTVDKSRWQEGNVFSCSVMHEALHNHYT QKSLSLSLGK | 11 |
| Light chain strand ([Anti-TIGIT light chain (kappa)]-[Linker (V-2)]-[IL-10]-[D-1]-[IL-10]) | DIVMTQSHSSLSASVGDRVSITCKASQDVSNTVAWY QQKPGQPPKPLIYSASYRYTGVPDRFTGSGSGTDFTL TISSLQAEDVAVYYCQQHYSTPWTFGGGTKVEIKRT VAAPSVFIFPPSDEQLKSGTASVVCLLNNFYPREAKV QWKVDNALQSGNSQESVTEQDSKDSTYSLSSTLTLS KADYEKHKVYACEVTHQGLSSPVTKSFNRGECGGG GSGGGGSGGGGSSPGQGTQSENSCTHFPGNLPNMLR DLRDAFSRVKTFFQMKDQLDNLLLKESLLEDFKGYL GCQALSEMIQFYLEEVMPQAENQDPDIKAHVNSLGE NLKTLRLRLRRCHRFLPCENKSKAVEQVKNAFNKLQ EKGIYKAMSEFDIFINYIEAYMTMKIRNGGGSGGGSP GQGTQSENSCTHFPGNLPNMLRDLRDAFSRVKTFFQ MKDQLDNLLLKESLLEDFKGYLGCQALSEMIQFYLE EVMPQAENQDPDIKAHVNSLGENLKTLRLRLRRCH RFLPCENKSKAVEQVKNAFNKLQEKGIYKAMSEFDI FINYIEAYMTMKIRN | 25 |

| **IL-10 dimer (2) is linked without a linker to each C-terminus of the heavy chain (2) of the anti-TIGIT antibody MMB0** | | |
|---|---|---|
| Heavy chain strand ([anti-TIGIT heavy chain (IgG1)]-[linker (V-1)]-[IL-10]-[D-1]-[IL-10]) | QVQLQESGPGLVKPSQTLSLTCTVTGYSITSDYAWN WIRQFPGNKLEWMAYITYSGSTSYNPSLKSRVTISVD TSKNQFSLKLSSVTAADTAVYYCASRQVGLGFAYWG QGTLVTVSSASTKGPSVFPLAPCSRSTSESTAALGCL VKDYFPEPVTVSWNSGALTSGVHTFPAVLQSSGLYSL SSVVTVPSSSLGTKTYTCNVDHKPSNTKVDKRVESK YGPPCPPCPAPEFLGGPSVFLFPPKPKDTLMISRTPEV TCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL TCLVKGFYPSDIAVEWESNGQPENNYKTTPPVLDSD GSFFLYSRLTVDKSRWQEGNVFSCSVMHEALHNHYT QKSLSLSLGKSPGQGTQSENSCTHFPGNLPNMLRDL RDAFSRVKTFFQMKDQLDNLLLKESLLEDFKGYLGC QALSEMIQFYLEEVMPQAENQDPDIKAHVNSLGENL KTLRLRLRRCHRFLPCENKSKAVEQVKNAFNKLQEK GIYKAMSEFDIFINYIEAYMTMKIRNGGGSGGGSPGQ GTQSENSCTHFPGNLPNMLRDLRDAFSRVKTFFQMK DQLDNLLLKESLLEDFKGYLGCQALSEMIQFYLEEV MPQAENQDPDIKAHVNSLGENLKTLRLRLRRCHRFL PCENKSKAVEQVKNAFNKLQEKGIYKAMSEFDIFIN YIEAYMTMKIRN | 26 |
| Light chain strand (Anti- | DIVMTQSHSSLSASVGDRVSITCKASQDVSNTVAWY QQKPGQPPKPLIYSASYRYTGVPDRFTGSGSGTDFTL TISSLQAEDVAVYYCQQHYSTPWTFGGGTKVEIKRT | 13 |
| TIGIT light chain (kappa)) | | |

| **IL-10 dimer (2) is linked via linker V-1 to each C-terminus of the heavy chain (2) of the anti-TIGIT antibody MMB0 (MMB3)** | | |
|---|---|---|
| Heavy chain strand ([anti-TIGIT heavy chain (IgG1)]-[linker (V-1)]-[IL-10]-[D-1]-[IL-10]) | QVQLQESGPGLVKPSQTLSLTCTVTGYSITSDYAWN WIRQFPGNKLEWMAYITYSGSTSYNPSLKSRVTISVD TSKNQFSLKLSSVTAADTAVYYCASRQVGLGFAYWG QGTLVTVSSASTKGPSVFPLAPCSRSTSESTAALGCL VKDYFPEPVTVSWNSGALTSGVHTFPAVLQSSGLYSL SSVVTVPSSSLGTKTYTCNVDHKPSNTKVDKRVESK YGPPCPPCPAPEFLGGPSVFLFPPKPKDTLMISRTPEV TCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL TCLVKGFYPSDIAVEWESNGQPENNYKTTPPVLDSD GSFFLYSRLTVDKSRWQEGNVFSCSVMHEALHNHYT QKSLSLSLGKEAAAKEAAAKEAAAKSPGQGTQSEN SCTHFPGNLPNMLRDLRDAFSRVKTFFQMKDQLDN LLLKESLLEDFKGYLGCQALSEMIQFYLEEVMPQAE NQDPDIKAHVNSLGENLKTLRLRLRRCHRFLPCENK SKAVEQVKNAFNKLQEKGIYKAMSEFDIFINYIEAY MTMKIRNGGGSGGGSPGQGTQSENSCTHFPGNLPN MLRDLRDAFSRVKTFFQMKDQLDNLLLKESLLEDFK GYLGCQALSEMIQFYLEEVMPQAENQDPDIKAHVN SLGENLKTLRLRLRRCHRFLPCENKSKAVEQVKNAF NKLQEKGIYKAMSEFDIFINYIEAYMTMKIRN | 27 |
| Light chain strand (Anti-TIGIT light chain (kappa)) | DIVMTQSHSSLSASVGDRVSITCKASQDVSNTVAWY QQKPGQPPKPLIYSASYRYTGVPDRFTGSGSGTDFTL TISSLQAEDVAVYYCQQHYSTPWTFGGGTKVEIKRT VAAPSVFIFPPSDEQLKSGTASVVCLLNNFYPREAKV QWKVDNALQSGNSQESVTEQDSKDSTYSLSSTLTLS KADYEKHKVYACEVTHQGLSSPVTKSFNRGEC | 13 |

| **IL-10 dimer (2) is linked via linker V-2 to each C-terminus of the light chain (2) of the anti-TIGIT antibody MMB0 (MMB4)** | | |
|---|---|---|
| Heavy chain strand ([anti-TIGIT heavy chain (IgG1)]-[linker (V-2)]-[IL-10] -[D-1]-[IL-10]) | QVQLQESGPGLVKPSQTLSLTCTVTGYSITSDYAWN WIRQFPGNKLEWMAYITYSGSTSYNPSLKSRVTISVD TSKNQFSLKLSSVTAADTAVYYCASRQVGLGFAYWG QGTLVTVSSASTKGPSVFPLAPCSRSTSESTAALGCL VKDYFPEPVTVSWNSGALTSGVHTFPAVLQSSGLYSL SSVVTVPSSSLGTKTYTCNVDHKPSNTKVDKRVESK YGPPCPPCPAPEFLGGPSVFLFPPKPKDTLMISRTPEV TCVVVDVSQEDPEVQFNWYVDGVEVHNAKTKPRE EQFNSTYRVVSVLTVLHQDWLNGKEYKCKVSNKGL PSSIEKTISKAKGQPREPQVYTLPPSQEEMTKNQVSL TCLVKGFYPSDIAVEWESNGQPENNYKTTPPVLDSD GSFFLYSRLTVDKSRWQEGNVFSCSVMHEALHNHYT QKSLSLSLGKGGGGSGGGGSGGGGSSPGQGTQSENS CTHFPGNLPNMLRDLRDAFSRVKTFFQMKDQLDNL LLKESLLEDFKGYLGCQALSEMIQFYLEEVMPQAEN QDPDIKAHVNSLGENLKTLRLRLRRCHRFLPCENKS KAVEQVKNAFNKLQEKGIYKAMSEFDIFINYIEAYM TMKIRNGGGSGGGSPGQGTQSENSCTHFPGNLPNML RDLRDAFSRVKTFFQMKDQLDNLLLKESLLEDFKGY LGCQALSEMIQFYLEEVMPQAENQDPDIKAHVNSLG ENLKTLRLRLRRCHRFLPCENKSKAVEQVKNAFNKL QEKGIYKAMSEFDIFINYIEAYMTMKIRN | 28 |
| Light chain strand (Anti-TIGIT light chain (kappa)) | DIVMTQSHSSLSASVGDRVSITCKASQDVSNTVAWY QQKPGQPPKPLIYSASYRYTGVPDRFTGSGSGTDFTL TISSLQAEDVAVYYCQQHYSTPWTFGGGTKVEIKRT VAAPSVFIFPPSDEQLKSGTASVVCLLNNFYPREAKV QWKVDNALQSGNSQESVTEQDSKDSTYSLSSTLTLS KADYEKHKVYACEVTHQGLSSPVTKSFNRGEC | 13 |

(In Table 7, the IL-10 dimer is formed by linking the IL-10 of SEQ ID NO. 9 through a D-1 linker (GGGSGGG; SEQ ID NO. 16))

Hereinafter, the above-described anti-TIGIT antibody fusion proteins with MMB0 and IL-10 (see Table 6) were named as shown in Table 8 below:

**[Table 8]**

| Description of the fusion protein | Name |
|---|---|
| IL-10 monomer (2) is linked via flexible linker V-2 to each C-terminus of the heavy chain (2) of the anti-TIGIT antibody MMB0 | MMB1 |
| IL-10 monomer (2) is linked via flexible linker V-2 to each C terminus of the light chains (2) of the anti-TIGIT antibody MMB0 | MMB2 |
| IL-10 dimer (2) is linked via rigid linker V-1 to each C-terminus of the heavy chain (2) of the anti-TIGIT antibody MMB0 | MMB3 |
| IL-10 dimer (2) is linked via flexible linker V-2 to each C-terminus of the light chain (2) of the anti-TIGIT antibody MMB0 | MMB4 |
| IL-10 dimer (2) is linked via rigid linker V-1 to each C terminus of the light chains (2) of the anti-TIGIT antibody MMB0 | MMB5 |
| IL-10 dimer (2) is linked via flexible linker V-2 to each C-terminus of the light chain (2) of the anti-TIGIT antibody MMB0 | MMB6 |

Hereinafter, the fusion protein of the anti-TIGIT antibody MMB0 with IL-10 prepared in the present Example are listed as named in Table 8 above.

### Example 3. Protein binding activity of MMB0-IL-10 fusion protein

### 3.1. TIGIT binding affinity

Recombinant human TIGIT protein (Acro Biosystems-Cat# TIT-H52H5) was diluted to a concentration of 1 mg/ml in coating buffer and coated onto ELISA plates (using an ELISA buffer kit (Invitrogen^{™}, CNB0011)). Coating was performed by incubating at 37°C for 2 hours. The plates were blocked with blocking buffer (1X assay buffer) for 1 hour, and anti-TIGIT monoclonal antibody (MMB0), MMB0-IL-10 fusion protein (MMB5), or human IgG4 (Biolegend, Cat#403701) were serially diluted starting from 62.5 nM in a 1:3 ratio using the assay buffer. The serially diluted drugs were placed onto the TIGIT-coated plates and incubated. Subsequently, anti-Human IgG-HRP antibody (Promega, Cat#W4031) diluted 1:10000 was added to each well. The plates were induced with TMB (tetramethylbenzidine) solution, and the reaction was stopped using ELISA stop solution (Invitrogen, Cat#SS04). OD values were measured at a wavelength of 450 nM using a GloMax^{®} Explorer.

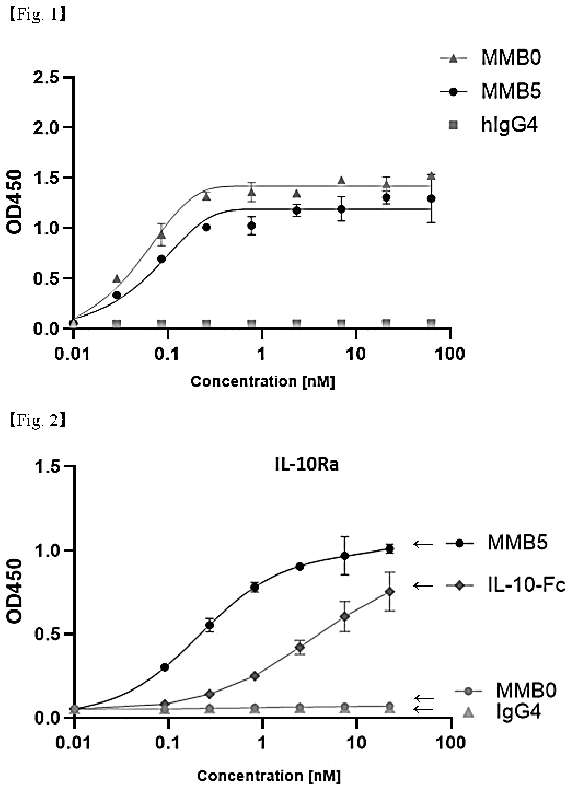

The binding affinities of the anti-TIGIT antibody (MMB0) and MMB0-IL-10 fusion protein (MMB5) for TIGIT are shown in Fig. 1. Fig. 1 shows that the MMB0-IL-10 fusion protein (MMB5) has an affinity for human TIGIT similar to that of the parental antibody (MMB0). These results demonstrate that the addition (fusion) of IL-10 to the MMB0-IL-10 fusion protein MMB5 does not interfere with the TIGIT binding site of the parental antibody (MMB0) or inhibit its binding ability, and that the fusion protein maintains the functional quality of the parental antibody.

### 3.2. Binding affinity for IL-10 receptor

Recombinant human IL-10RA His-tag Protein (Sino Biological, 10419-H08H) was diluted to a concentration of 0.1 µg/ml using coating buffer B (Invitrogen, CB01100) and coated onto ELISA plates. 200 µl of 1X Assay Buffer (Invitrogen, CNB0011) was added to each well and blocked by incubation at room temperature (RT) for 1 hour. 100 µl each of the samples (anti-TIGIT monoclonal antibody (MMB0), MMB0-IL-10 fusion protein (MMB5), human IL-10-Fc (Human IgG1 AdipoGen, Cat#CHI-HF-22010), and human IgG4 (Biolegend, Cat#403701)) were added to the blocked plate and incubated at 37°C for 1 hour with shaking (200 rpm). The above samples were each diluted with assay buffer to a concentration of 200 nM. The detection antibody (HRP-conjugated anti-Human IgG (H+L) antibody (Promega, Cat#W4031)) was diluted in assay buffer at a ratio of 1:10000, 100 µl was added to each well, and the plate was incubated at 37°C for 1 hour with shaking (200 rpm). Subsequently, 100 µl of TMB solution was added to each well. After adding and inducing luminescence, 100 µl of ELISA Stop solution (Invitrogen, Cat#SS04) was added to stop the reaction. The OD value was measured at a wavelength of 450 nM using a GloMax^{®} Explorer.

The binding affinity of the anti-TIGIT antibody (MMB0) and fusion protein (MMB5) for IL-10Ra is shown in Fig. 2. As shown in Fig. 2, the parental antibody MMB0 did not bind to IL-10RA, whereas the fusion protein (MMB5), in which IL-10 is linked to the anti-TIGIT antibody, showed binding affinity for IL-10Ra and exhibited a stronger binding affinity for IL-10 than IL-10-Fc.

### Example 4. Cell binding activity of fusion protein

### 4.1. Binding Affinity of TIGIT on CD4+ T Cells, CD8+ T Cells, and CD56+ NK Cells

In this Example, the binding ability of the fusion protein (MMB5) to each cell populations of immune cells (CD4+ T Cell, CD8+ T Cell, and CD56+ NK Cell) expressing TIGIT was tested using an anti-TIGIT antibody labeled with PE/Cy7. Since the fusion protein (MMB5) binds competitively to TIGIT expressed on the surface of the immune cells, including the anti-TIGIT antibody labeled with PE/Cy7, as the fusion protein (MMB5) binds to the immune cells through TIGIT expressed on the surface of the immune cells, the binding percentage of the anti-TIGIT antibody labeled with PE/Cy7 to TIGIT on the surface of the immune cells will decrease. Therefore, the binding ability of the fusion protein (MMB5) to the immune cells was tested by measuring the degree of decrease in the binding percentage of the anti-TIGIT antibody labeled with PE/Cy7.

Peripheral blood mononuclear cells (PBMCs; STEMCELL Technologies) from healthy human donors were counted and resuspended at a rate of 5 million cells per 1 ml of PBS (performed in a SPL, Cat#34296, 96-well plate (round bottom)). The prepared cells (PBMCs) were bound with MMB0-IL-10 fusion proteins MMB1, MMB3, and MMB5 (68.7 nM each) or their parental antibody MMB0 (68.7 nM) in a 37°C incubator for 15 minutes. The plate was centrifuged at 1500 rpm for 3 minutes at room temperature to remove unbound antibodies, and then stained with an antibody cocktail. The above antibody cocktail consisted of anti-CD3 APC (Biolegend, 1:50), anti-CD4 AF700 (Biolegend, 1:50), anti-CD8 FITC (Biolegend, 1:50), anti-CD56 BV421 (Biolegend, 1:50), and anti-hTIGIT PE/Cy7 (Biolegend, 1:50). After performing the staining in the dark at room temperature for 10 minutes, 100 µl of PBS was added, and the cells were centrifuged at 1500 rpm for 3 minutes at room temperature for washing. Subsequently, the supernatant was removed, and the cells were resuspensed with 100 µl of PBS. Then, flow cytometry was performed using CytoFLEX (Beckman Coulter) at a rate of 60 µl/min (abort rate ≤ 5%). Accordingly, the level of TIGIT bound to the above-mentioned anti-hTIGIT PE/Cy7 Antibody (anti-hTIGIT Antibody labeled with PE/Cy7) can be measured. For comparison, the same test was performed on an unstimulated group that was not treated with the fusion protein or the parent antibody.

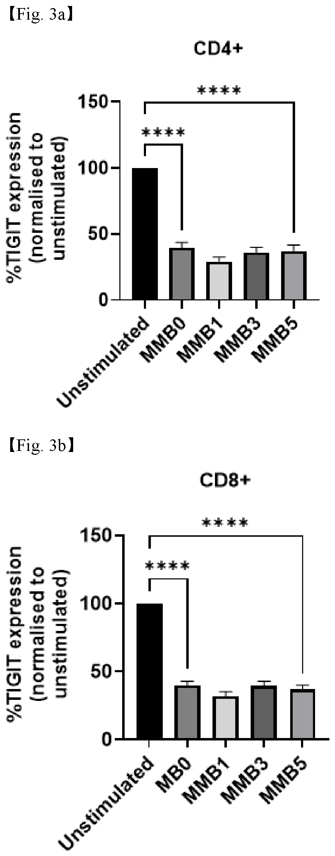

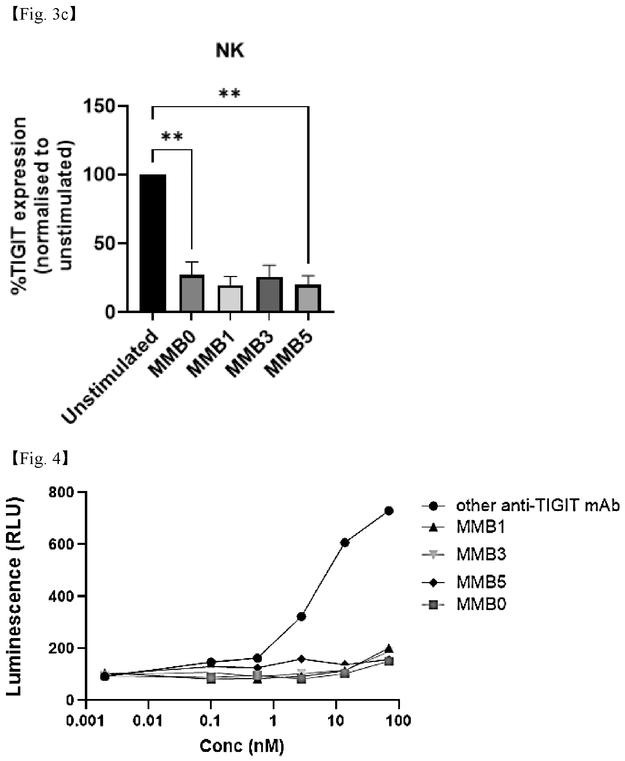

The proportion of TIGIT on the surface of immune cells labeled by binding to the anti-hTIGIT PE/Cy7 antibody ("indicated as % TIGIT expression") is shown in Figs. 3a to 3c (3a: CD4+ T cell; 3b: CD8+ T cell; 3c: NK cell).

As previously explained, the lower the level of TIGIT labeled by the binding of the anti-hTIGIT PE/Cy7 Antibody, the higher the cell binding affinity through TIGIT of the treated sample; therefore, the results of Fig. 3a to 3c show that the anti-TIGIT Antibody-IL-10 fusion protein or parental Antibody (anti-TIGIT Antibody) provided in the present disclosure binds to effector T cells and NK cells with excellent binding affinity.

### Example 5. Maintaining TIGIT/CD155 interaction/signaling functions

In this Example, TIGIT/CD155 Blockade was tested according to Promega's TIGIT/CD155 Blockade Bioassay instructions to determine whether the MMB0-IL-10 fusion protein provided in the present disclosure maintains TIGIT/CD155 interaction/signaling function. More specifically, TIGIT effector cells (Jurkat cell lines expressing Human TIGIT; Promega) were thawed, transferred to assay buffer (Promega), seeded in a white 96-well plate, and rested in an incubator for one day. On the next day, MMB0-IL-10 fusion proteins MMB1, MMB3, MMB5, and their parent antibody MMB0 were treated with the concentration described in Fig. 4. Subsequently, CD155-CHO-K1 (Promega) was added to all experimental groups, including a negative control group (a group not treated with the drug (fusion protein or antibody)). Subsequently, the samples were incubated in a 37°C incubator for 6 hours, followed by leaving them at room temperature for 10-15 minutes. Finally, Bio-Glo^{™} Reagent was added to all experimental groups, including the negative control, and after incubation at room temperature, luminescence was measured three times using a luminescence plate reader (using a luminescence plate reader (BHS LumiStar)).

For comparison, the same experiment was performed using an antagonistic anti-TIGIT antibody (indicated as "other anti-TIGIT mAb" in Fig. 4) as the control antibody. The sequence information of the control used is as follows:
Heavy chain: 
QVQLQESGPGLVKPSQTLSLTCTVTGYSITSDYAWNWIRQPPGKGLEWM
GYISYSGSARYNPSLKSRVTISRDTSKNQFSLKLSSVTAEDTATYYCARKGYPAY
FAYWGQGTLVTVSSASTKGPSVFPLAPSSKSTSGGTAALGCLVKDYFPEPVTVS
WNSGALTSGVHTFPAVLQSSGLYSLSSVVTVPSSSLGTQTYICNVNHKPSNTKVD
KRVEPKSCDKTHTCPPCPAPELLGGPSVFLFPPKPKDTLMISRTPEVTCVVVDVS
HEDPEVKFNWYVDGVEVHNAKTKPREEQYNSTYRVVSVLTVLHQDWLNGKE
YKCKVSNKALPAPIEKTISKAKGQPREPQVYTLPPSREEMTKNQVSLTCLVKGF
 
YPSDIAVEWESNGQPENNYKTTPPVLDSDGSFFLYSKLTVDKSRWQQGNVFSCS
VMHEALHNHYTQKSLSLSPGK (SEQ ID NO: 43)
Light chain:

DIQMTQSPSSLSASVGDRVSITCKASQDVSTAVAWYQQKPGQAPRLLIYS
ASYRYTGVPDRFSGSGSGTDFTLTISSLQPEDFATYYCQHHYSTPYTFGQGTKLEI
KRTVAAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNRGE
C (SEQ ID NO: 44)

The results obtained above are shown in Fig. 4. As shown in Fig. 4, the MMB0-IL-10 fusion proteins MMB1, MMB3, MMB5, and their parental antibody MMB0 did not block the interaction (binding) between TIGIT and CD155. This demonstrates that the fusion protein provided in the present application can utilize the benefits of CD155 signaling that naturally occurs in the immune microenvironment by immune cells, and can also utilize the advantages of the TIGIT-stimulated signal of the included anti-TIGIT monoclonal antibody.

### Example 6. Induction of IL-10R (IL-10 receptor) signaling (measured by STAT3 phosphorylation)

The immunostimulatory effect of MMB0-IL-10 fusion protein was tested in human CD3+ T cells (CD4+ and CD8+ T cells) using STAT3 phosphorylation assay.

PBMCs (STEMCELL Technologies) from healthy human donors were seeded into plates at a concentration of 5 x 10⁵ cells/well. The cells were treated with 68.7 nM concentrations of the drug (MMB0-IL-10 fusion protein MMB1, MMB2, MMB3, MMB4, MMB5, MMB6) and the control drug of human IL-10 (Miltenyi Biotec, 130-093-948), respectively, and stimulated in a 37°C incubator for 15 minutes. After centrifugation (1500 rpm, 3 min, 4°C), the supernatant was removed, and the cells were stained with 50 µl of surface marker antibody at 4°C for 10 minutes. The surface marker antibodies refer to Live/Dead-AV750 (eBioscience, 65-0865-12, 1:500), anti-CD3-BV150 (Biolegend, 344828, 1:50), anti-CD4-A700 (Biolegend, 344622, 1:50), and anti-CD8-FITC (Biolegend, 980908, 1:50), and these markers were used after diluting them to the respective ratios listed in PBS. Subsequently, after removing the supernatant by centrifugation (1500 rpm, 3 min, 4°C), 100 µl of Fix buffer (Biolegend, 420801) was added to the cells, and the cells were reacted at room temperature for 15 minutes. After the reaction was complete, the cells were centrifuged (1500 rpm, 3 min, room temperature) and the supernatant was removed. Then, 100 µl of True-Phos perm buffer (Biolegend, 425401) was added to the cells, and the reaction was carried out at -20°C for 1 hour. Subsequently, the cells were centrifuged (1500 rpm, 3 min) to remove the supernatant and stained with pSTAT3 antibody. For the staining, pSTAT3-APC (Biolegend, 651008, 1:50) antibody was diluted in PBS and the process was performed under light-blocking conditions. After staining, the cells were centrifuged (1500 rpm, 3 min) to remove the supernatant, and then 100 µl of PBS was added to resuspend the cells. The fluorescence of the antibody detecting surface marker labeling and phosphorylated STAT3 (pSTAT3) of each cell was detected using a cell analyzer (CytoFLEX, Beckman Coulter). The data was analyzed using Flowjo software (BD Biosciences) and plotted using GraphPad Prism 9 software (Dotmatics).

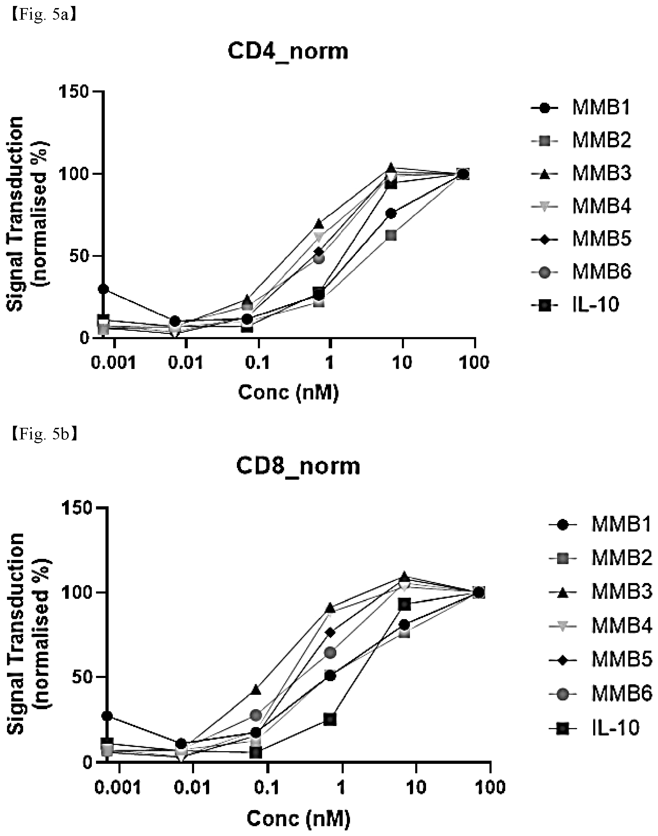

The results obtained above (signaling transduction) are shown in Fig. 5a and 5b (5a: CD4+ cell; 5b: CD8+ cell; the results are obtained by normalizing the signal for each concentration with the signal at each drug concentration of 68.7 nM set to 100%).

As shown in Figs. 5a and 5b, phosphorylation of STAT3 in CD4+ T cells and CD8+ T cells was observed to be high at 68.7 nM for all tested fusion proteins, and in the case of IL-10 (human IL-10 in its free form but not in the fusion protein; control drug), it was found that it began to lose binding efficiency to IL-10R at 0.687 nM. This experiment suggests that the MMB fusion antibody can induce pSTAT3 signaling at lower concentrations compared to free hIL-10, and that IL-10 bound to the antibody sustains signal transduction.

### Example 7. IL-10 delivery

In this Example, the effect of the MMB0-IL-10 fusion protein provided in the present disclosure for transporting IL-10 to immune cells (effector T cells, NK cells) was tested.

PBMC (STEMCELL Technologies) obtained from a healthy human donor was seeded into a 96-well plate at a concentration of 2 x 10⁵ cells/well, and then MMB0-IL-10 fusion protein MMB1 was injected and reacted in a 37°C incubator for 15 minutes. Afterward, the cells were centrifuged (1500 rpm, 3 minutes), the supernatant was removed, 50 µl of antibody master mix was added to the cells to resuspend them, and the reaction was carried out in the dark. The above antibody master mix is prepared by diluting anti-CD3-BV510 (Biolegend, 344828, 1:50), anti-CD4-AF700 (Biolegend, 344622, 1:50), anti-CD8-FTIC (Biolegend, 980908, 1:50), anti-CD56-BV421 (Biolegend, 318328, 1:50), anti-IL-10-PE (Miltenyi Biotec, 130-112-728, 1:50), and Live/Dead-A750 (ebioscience, 1:50) in PBS buffer. After centrifuging the obtained reactant (1500 rpm, 3 min), the supernatant was removed and the solution was resuspended in 100 µl of PBS. Fluorescence values were measured using a cell analyzer (CytoFLEX, Beckman Coulter), and the ratio of cells with PE (Phycoerythrin) color was measured. The ratio of cells with PE color represents the ratio of cells bound (transported) to IL-10. The data were analyzed using Flowjo software (BD Biosciences) and plotted using GraphPad Prism 9 software (Dotmatics).

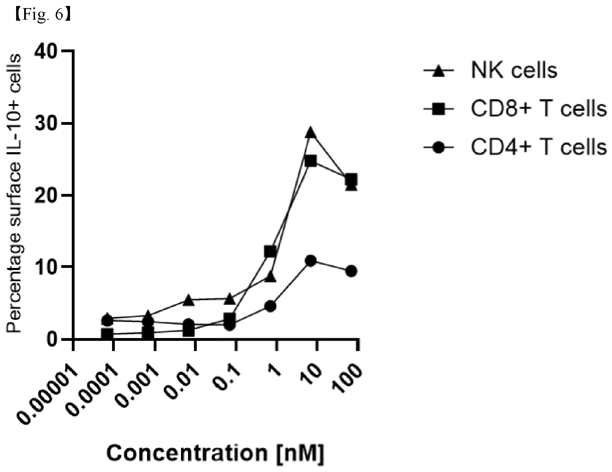

The results obtained above (the ratio of cells bound (transported) to IL-10) are shown in Fig. 6. As shown in Fig. 6, IL-10 binding increases up to a concentration of 6.87 nM of MMB0-IL-10 fusion protein (MMB1) and then decreases slightly at 68.7 nM. This suggests that the MMB0-IL-10 fusion protein provided in the present disclosure can deliver IL-10 to effector T cells and NK cells in proportion to the concentration.

### Example 7. Suppressive function (in vitro)

### 7.1. Inhibition of Pro-Inflammatory Cytokines Production

In this Example, the inhibitory effect of the MMB0-IL-10 fusion proteins provided in the present disclosure on the production of pro-inflammatory cytokines was tested.

PBMCs from healthy human donors (STEMCELL Technologies; 2 x 10⁵ cells/well) were stimulated for 72 hours using anti-CD3 antibody (BD, 1 µg/ml), anti-CD28 antibody (BD, 0.00032 µg/ml), and drugs. The drugs referred to here are MMB0-IL-10 fusion proteins MMB3 and MMB5, and their parent antibody, MMB0. Each drug was stimulated at 37°C with a concentration of 68.7 nM. Subsequently, LegendPlex was performed on the supernatant to measure pro-inflammatory cytokines TNF-α and Granzyme B. For comparison, the same test was performed using anti-CD3 antibody/anti-CD28 antibody without the aforementioned drug treatment (indicated as 'anti-CD3/CD28').

LegendPlex was performed according to the manufacturer's manual using LEGENDplex^{™} Buffer set A (Biolegend, 740368), LEGENDplex^{™} Human CD8/NK Panel Standard (Biolegend, 740281), LEGENDplex^{™} Human CD8/NK Panel Detection Antibodies (Biolegend, 740282), LEGENDplex^{™} Human TNF-α Capture Bead A10 (Biolegend, 740273), and LEGENDplex^{™} Human Granzyme B Capture Bead B6 (Biolegend, 740278).

More specifically, the supernatant was diluted 1:1 with assay buffer (Invitrogen) and mixed with beads. The mixture was then diluted in a shaker at 8000 rpm for 2 hours at room temperature under light blockage. After centrifugation (1050 rpm, 5 min), the supernatant was completely removed, wash buffer was added, and the mixture was incubated at room temperature (RT) for 1 minute. Subsequently, the supernatant was removed by centrifugation (1050 rpm, 5 min). Then, the detection antibody was added and the mixture was shaken at room temperature for 1 hour under light blockage. Afterward, SA-PE (Streptavidin-Phycoerythrin Conjugate) was added, and the mixture was shaken at room temperature for 30 minutes under light blockage. Finally, the mixture was centrifuged (1050 rpm, 5 min) and the supernatant was removed. 150 µl of wash buffer was added for resuspension, and TNF-α and Granzyme B were quantified by measuring fluorescence using LegendPlex.

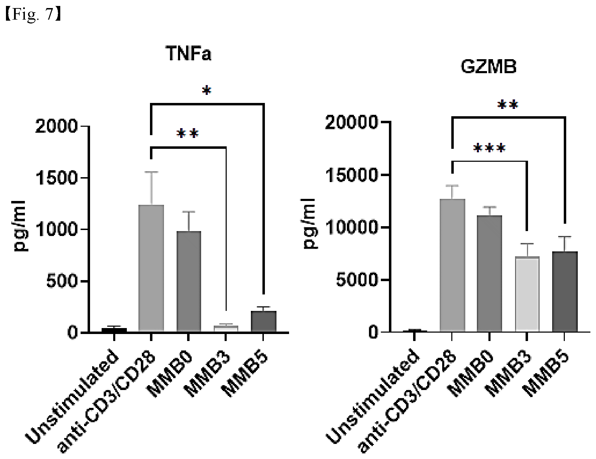

The results obtained above are shown in Fig. 7. As shown in Fig. 7, all tested MMB0-IL-10 fusion proteins reduced TNF-α and Granzyme (GZMB) of pro-inflammatory cytokines that were increased by anti-CD3/CD28 stimulation. These results suggest that the MMB0-IL-10 fusion protein provided in the present disclosure maintains the anti-inflammatory efficacy of IL-10.

### 7.2. Inhibition of T Cell Proliferation

In this Example, the inhibitory effect of the MMB0-IL-10 fusion proteins provided in the present disclosure on T cell proliferation was tested.

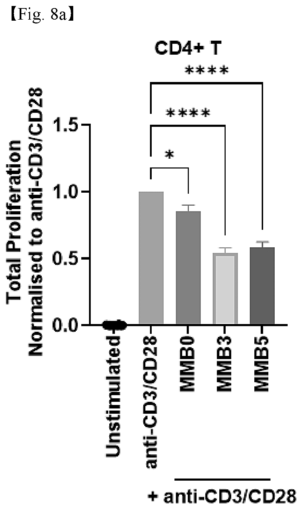

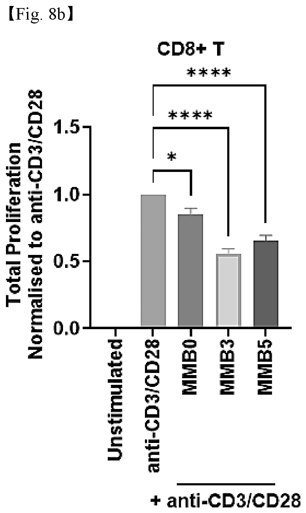

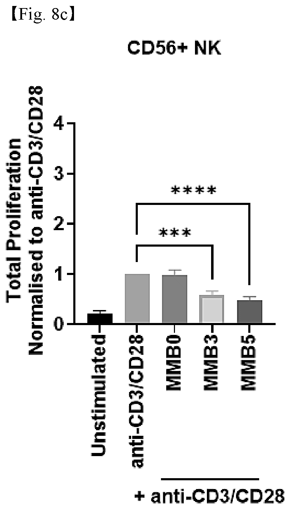

Healthy human donor PBMCs (STEMCELL Technologies) labeled with CellTrace Violet (Invitrogen, C34557) were stimulated for 72 hours using anti-CD3 antibody (BD, 0.1 µg/ml), anti-CD28 antibody (BD, 0.00032 µg/ml), and drugs at a concentration of 2 x 10⁶ cells/ml. Here, the drugs refer to MMB0-IL-10 fusion proteins MMB3 and MMB5, and their parent antibody, MMB0, with each drug concentration set to 68.7 nM. Subsequently, the plates were centrifuged (400 g, 3 min, room temperature) to remove the supernatant. Cells were cultured in Viability Dye eFluor 780 (1/1000) diluted in PBS at room temperature for 10 minutes in the dark. After centrifugation (400 g, 3 min, room temperature) The supernatant was removed. The obtained cells were made permeable by shielding them from light in FOXP3 Perm buffer (eBioscience) at room temperature for 15 minutes. After centrifugation (400g, 3min, room temperature), the supernatant was removed, and the cells were stained with internal staining antibodies. Internal staining antibodies refer to AF700 anti-CD4 (Biolegend, 344622, 1/50), APC anti-Ki67 (Biolegend, 350504, 1/50), BV510 anti-CD3 (Biolegend, 344828, 1/50), PE anti-CD56 (Biolegend, 318306, 1/50), and PerCP anti-CD8 (Biolegend, 980916, 1/50). After centrifugation and supernatant removal, 100µl of PBS was added and the cells were resuspended. Fluorescence values were measured using an analyzer (CytoFLEX, Beckman Coulter). Data were analyzed using Flowjo software (BD Biosciences) and plotted using GraphPad Prism 9 software (Dotmatics). For comparison, the same test was performed using anti-CD3 antibody/anti-CD28 antibody without the aforementioned drug treatment (indicated as 'anti-CD3/CD28' in Figs. 8a-8c).

The results obtained above are shown in Fig. 8a (CD4+ T cell), 8b (CD8+ T cell), and 8c (NK cell). As shown in Figs. 8a-8c, it can be seen that all tested MMB0-IL-10 fusion proteins inhibit the proliferation of CD4+ T cells, CD8+ T cells, and NK cells induced by stimulation of the anti-CD3/CD28 antibody. On the other hand, the parental antibody (MMB0) was found to have weak or low statistical significance in this inhibitory ability. Through this, it can be seen that the inhibitory effect on T cell and NK cell proliferation is due to IL-10 signaling rather than TIGIT signaling by the MMB0-IL-10 fusion protein. In conclusion, the MMB0-IL-10 fusion proteins provided in the present disclosure suggest that they can inhibit the proliferative effects of anti-CD3/CD28 on CD4+ T cells, CD8+ T cells, and NK cells.

### 7.3. Inhibition of production (expression) of pro-inflammatory cytokines in PBMCs derived from patients with autoimmune diseases

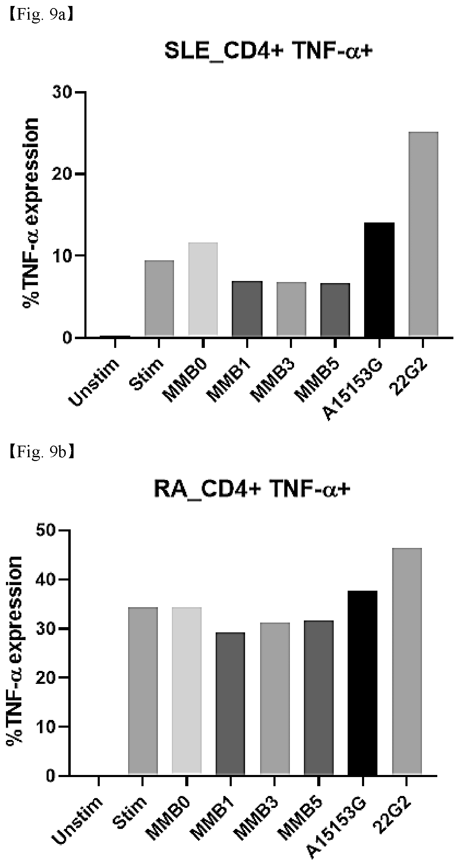

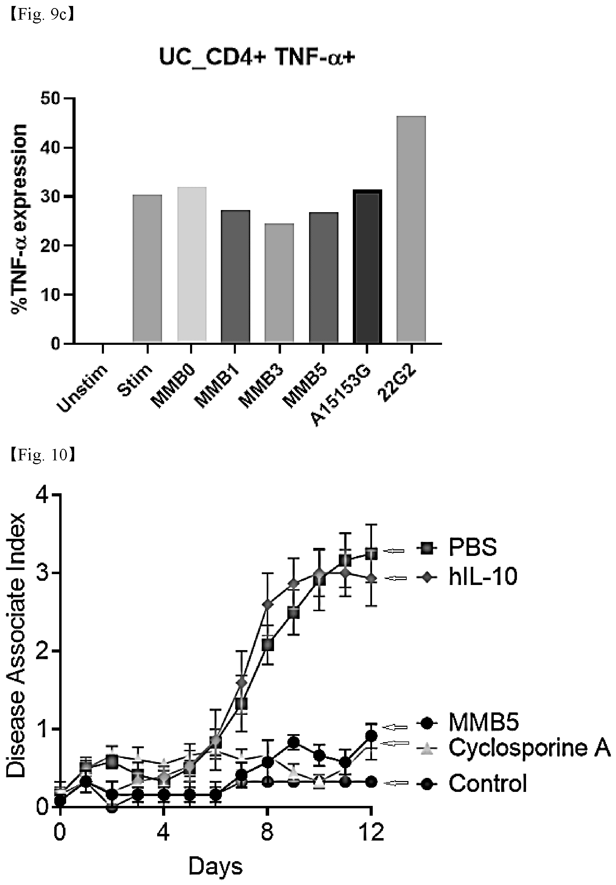

PBMC (STEMCELL Technologies) from patients with autoimmune diseases such as systemic lupus erythematosus (SLE), rheumatoid arthritis (RA), and ulcerative colitis (UC) were inoculated into a concentration of 5 x 10⁵ cells/well. Cells were stimulated using Anti-CD3 Antibody (BD, 555336, 1 µg/ml) and Anti-CD28 (BD, 555725, 1 µg/ml), and drugs were added at 68.7 nM. Here, the drugs refer to MMB0-IL-10 fusion proteins MMB1, MMB3, and MMB5, their parent antibody MMB0, and control anti-TIGIT antibodies A15153G (Biolegend, 372702) and 22G2 (BPS, 71340). After treating with the drug and incubating in a 37°C, 5% CO2 incubator for 4 hours, Brefeldin A (Sigma-Aldrich, B7651) was added at a concentration of 10 µg/ml to prevent cytokine release, and the cells were incubated in a 37°C incubator for 15 hours. In conclusion, the cells (PBMCs derived from patients with autoimmune diseases) reacted with the drug and stimulant (anti-CD3/CD28) for 19 hours. After the reaction was completed, the supernatant was removed by centrifugation (1500 rpm, 5 min, 4°C), and Zombie Aqua Fixable dead cell dye solution (Biolegend, 77143) was added for Live/Dead staining. The cells were then incubated at room temperature for 20 minutes under light protection. After the reaction was finished, the supernatant was removed by centrifugation (1500 rpm, 5 min, 4°C), 150 µl of surface marker antibody master mix was added, and the mixture was stained in the dark at 4°C for 30 minutes. Surface marker antibody master mix refers to anti-CD3-BV421 (Biolegend, 300434, 1:100), anti-CD4-PerCP-Cy5.5 (Biolegend, 3446081:100), anti-CD8-AF700 (Biolegend, 301028, 1:200), and anti-CD56-APC (Biolegend, 362504, 1:100) diluted in FACS buffer (0.1% FBS containing PBS). After staining is finished, centrifuge (1500 rpm, 5 min, 4°C) to remove the supernatant, add 100 µl of Fix/perm buffer (eBioscience, 00-5523-00), and fix the cells at room temperature for 40 minutes in a light-shielded state. Afterward, centrifuge (1500 rpm, 5 min, 4°C), remove the supernatant, and stain with an intracellular staining antibody at room temperature for 30 minutes in a light-shielded state. The intracellular staining antibody refers to anti-TNF-α-PE-Cy7 (Biolegend, 502930, 1:50) diluted in Perm buffer (eBioscience, 00-5523-00). After staining, the supernatant was removed by centrifugation (1500 rpm, 5 min, 4°C), 200 µl of PBS was added, and the sample was suspended. Fluorescence values were measured using a Cell analyzer (CytoFLEX, Beckman Coulter). Data were analyzed using Flowjo software (BD Biosciences) and plotted using GraphPad Prism 9 software (Dotmatics). For comparison, the same test was performed using anti-CD3 antibody/anti-CD28 antibody without the aforementioned drug treatment (indicated as 'Stim' in Figs. 9a-9c).

The results obtained above (ratio of TNF-α-expressing CD4+ T cells) are shown in Fig. 9a (PBMC derived from SLE patients), 9b (PBMC derived from RA patients), and 9c (PBMC derived from UC patients). As shown in Figs. 9a-9c, stimulation by the anti-CD3/CD28 antibody increased the ratio of CD4+ T cells expressing TNF-α, a pro-inflammatory cytokine involved in the pathological symptoms of autoimmune diseases; conversely, this increased ratio of TNF-α-expressing CD4+ T cells was found to decrease upon treatment with all tested MMB0-IL-10 fusion proteins. This effect of reducing TNF-α-expressing CD4+ T cells by MMB0-IL-10 fusion proteins was observed in all PBMCs obtained from patients with the tested autoimmune diseases, namely Systemic Lupus Erythematosus (SLE), Rheumatoid Arthritis (RA), and Ulcerative Colitis (UC). In addition, the effect of reducing TNF-α-expressing CD4+ T cells by the MMB0-IL-10 fusion protein is superior compared to A15153G which is an antibody known to have anti-TIGIT agonistic function used as a control drug. With 22G2, which has anti-TIGIT antagonistic function used as a control drug, it can be seen that CD4+ T cells expressing TNF-α actually increase. Therefore, it can be seen that the MMB0-IL-10 fusion protein provided in the present disclosure has an excellent anti-inflammatory effect by reducing CD4+ T cells that produce the pro-inflammatory cytokine TNF-α in patients with various autoimmune diseases.

### Example 8. Anti-inflammatory effect (in vivo)

In this Example, the anti-inflammatory effect of MMB0-IL-10 fusion protein was tested by measuring the DAI score in a DSS-induced colitis model.

More specifically, human TIGIT KI balb/c mice with induced enteritis using 3% DSS (Dextran sulfate sodium) were observed for 11 days after administering the drugs via intraperitoneal or oral administration. The drugs referred to here were MMB0-IL-10 fusion protein MMB5 of 5 mg/kg, Cyclosporine A (MCE, 34526) of 40 mg/kg (a drug primarily used for inflammatory bowel disease (IBD)), and human IL-10 (Peprotech, 200-10) of 0.2 mg/kg as a control; Cyclosporine A was administered orally, while the other drugs were administered intraperitoneally. The DAI score (Disease Activity Index Score) was measured daily during the experiment and calculated according to the following formula (DAI score = (Body mass drop fraction + feces property score + fecal occupational blood score) / 3).

The results obtained above are shown in Fig. 10. As shown in Fig. 10, in the DSS-induced colitis animal model experiment, the group administered with soluble human IL-10 did not show an anti-inflammatory effect (reduction in DAI score), whereas MMB0-IL-10 fusion protein (MMB5) reduced the DAI score to a level similar to that of the positive control, Cyclosporine A, and showed higher anti-inflammatory efficacy than Cyclosporine A up to 8 days after administration. These results demonstrate that the MMB0-IL-10 fusion protein provided in the present disclosure has a potent anti-inflammatory effect against inflammatory diseases.

## Claims

1. An anti-TIGIT antibody or an antigen-binding fragment thereof, comprising:
a polypeptide comprising the amino acid sequence of SEQ ID NO: 1 (CDR-H1);
a polypeptide comprising the amino acid sequence of SEQ ID NO: 2 (CDR-H2);
a polypeptide comprising the amino acid sequence of SEQ ID NO: 3 (CDR-H3);
a polypeptide comprising the amino acid sequence of SEQ ID NO: 4 (CDR-L1);
a polypeptide comprising the amino acid sequence of SEQ ID NO: 5 (CDR-L2); and
a polypeptide comprising the amino acid sequence of SEQ ID NO: 6 (CDR-L3).

2. The anti-TIGIT antibody or antigen-binding fragment thereof of claim 1, comprising:
a heavy chain variable region comprising an amino acid sequence selected from SEQ ID NO: 7 and SEQ ID NOs: 29 to 34; and
a light chain variable region comprising an amino acid sequence selected from SEQ ID NO: 8 and SEQ ID NOs: 35 to 41.

3. The anti-TIGIT antibody or antigen-binding fragment thereof of claim 1, wherein the anti-TIGIT antibody is an animal antibody, a chimeric antibody, or a humanized antibody.

4. The anti-TIGIT antibody or antigen-binding fragment thereof of claim 1, wherein the antigen-binding fragment is an scFv, scFv-Fc, (scFv)2, Fab, Fab', or F(ab')₂ of the anti-TIGIT antibody.

5. A fusion protein comprising:
(1) an anti-TIGIT antibody or an antigen-binding fragment thereof; and
(2) interleukin-10 (IL-10).

6. The fusion protein of claim 5, wherein the anti-TIGIT antibody or antigen-binding fragment thereof comprises:
a polypeptide comprising the amino acid sequence of SEQ ID NO: 1 (CDR-H1);
a polypeptide comprising the amino acid sequence of SEQ ID NO: 2 (CDR-H2);
a polypeptide comprising the amino acid sequence of SEQ ID NO: 3 (CDR-H3);
a polypeptide comprising the amino acid sequence of SEQ ID NO: 4 (CDR-L1);
a polypeptide comprising the amino acid sequence of SEQ ID NO: 5 (CDR-L2); and
a polypeptide comprising the amino acid sequence of SEQ ID NO: 6 (CDR-L3).

7. The fusion protein of claim 5, wherein the anti-TIGIT antibody or antigen-binding fragment thereof comprises:
a heavy chain variable region comprising an amino acid sequence selected from SEQ ID NO: 7 and SEQ ID NOs: 29 to 34; and
a light chain variable region comprising an amino acid sequence selected from SEQ ID NO: 8 and SEQ ID NOs: 35 to 41.

8. The fusion protein of claim 5, wherein the IL-10 is a monomer or a dimer.

9. The fusion protein of claim 5, wherein the fusion protein does not comprise a peptide linker between the anti-TIGIT antibody or antigen-binding fragment thereof and interleukin-10.

10. The fusion protein of claim 5, wherein the fusion protein comprises a peptide linker between the anti-TIGIT antibody or antigen-binding fragment thereof and interleukin-10.

11. The fusion protein of claim 10, wherein the peptide linker is represented by [EAAAK]n or [(G)mS]l, wherein n is an integer from 1 to 5 representing the number of [EAAAK], m is an integer from 1 to 5 representing the number of G, and l is an integer from 1 to 5 representing the number of [(G)mS].

12. A nucleic acid molecule encoding the anti-TIGIT antibody or antigen-binding fragment thereof of any one of claims 1 to 4, or the fusion protein of any one of claims 5 to 11.

13. A recombinant vector comprising the nucleic acid molecule of claim 12.

14. A recombinant cell comprising the nucleic acid molecule of claim 12 or a recombinant vector comprising the same.

15. A pharmaceutical composition for preventing or treating an immune-related disease, comprising the anti-TIGIT antibody or antigen-binding fragment thereof of any one of claims 1 to 4, or the fusion protein of any one of claims 5 to 11.

16. The pharmaceutical composition for preventing or treating an immune-related disease of claim 15, wherein the immune-related disease is an autoimmune disease or an inflammatory disease.

17. The pharmaceutical composition for preventing or treating an immune-related disease of claim 16, wherein the autoimmune disease is rheumatoid arthritis, type 1 diabetes, Crohn's disease, ulcerative colitis, Behçet's syndrome, lupus, Sjögren's syndrome, myasthenia gravis, scleroderma, hypothyroidism, hyperthyroidism, psoriasis, vitiligo, multiple sclerosis, autoimmune hepatitis, autoimmune nephritis, autoimmune pancreatitis, autoimmune encephalitis, or cytokine storm.

18. The pharmaceutical composition for preventing or treating an immune-related disease of claim 16, wherein the inflammatory disease is inflammation or a disease caused by inflammation.

19. A pharmaceutical composition for immunosuppression, comprising the anti-TIGIT antibody or antigen-binding fragment thereof of any one of claims 1 to 4, or the fusion protein of any one of claims 5 to 11.
